# EUROPEAN PATENT APPLICATION

(11) **EP 3 597 042 A2**
(43) Date of publication of application: **22.01.2020**
(21) Application number: 18728434.4
(22) Date of filing: 14.03.2018
(51) Int. Cl.: A01N 63/00, A01N 65/00, A01N 25/00

(54) **METHODS AND COMPOSITIONS FOR IMPROVING PLANT HEALTH AND/OR YIELD**

(30) Priority: 14.03.2017 US 201762471084 P
(71) Applicant: Plantresponse Biotech, S.L., 28223 Pozuelo de Alarcón-Madrid (ES)
(72) Inventor: BORJA, Marisé, E-28224 Pozuelo de Alarcón-Madrid (ES); BRUNNER, Frédéric, 78464 Konstanz (DE); BONET GIGANTE, Julio, E-28031 Madrid (ES); OLIVARES, Patricia, E-28523 Rivas Vaciamadrid-Madrid (ES); SANZ, Yolanda, E-28041 Madrid (ES); PÉREZ, Rosa, E-28020 Madrid (ES)
(74) Representative: HGF Limited
(86) International application number: PCT/ES2018/070193
(87) International publication number: WO 2018/167347

(57) **Abstract**

The present invention is directed to methods and compositions for increasing a growth characteristic of a plant, increasing nutrient use efficiency of a plant, or improving a plant's ability to overcome biotic or abiotic stress comprising applying a composition comprising a fungal mycelia extract comprising piperidine and/or an analogue thereof (e.g., 6-oxopiperidine-2-carboxylic acid) and/or a salt thereof (e.g., 6-oxopiperidine-2-carboxylate), or any combination thereof to a plant, plant part, or to a propagation material of the plant.

## Description

### STATEMENT OF PRIORITY

This application claims the benefit, under 35 U.S.C. § 119 (e), of U.S. Provisional Application No. 62/471,084, filed on March 14, 2017, the entire contents of which are incorporated by reference herein.

### FIELD OF INVENTION

The present invention is relates to methods and compositions for increasing a growth characteristic of a plant, increasing nutrient use efficiency of a plant, or improving a plant's ability to overcome biotic or abiotic stress comprising applying a composition comprising a fungal mycelia extract comprising piperidine and/or an analogue thereof, and/or, a salt thereof, or any combination thereof to a plant, plant part, or to a propagation material of the plant.

### BACKGROUND

Plants are constantly challenged by biotic and abiotic stresses including microbial pathogens, such as bacteria, fungi, and viruses, and/or changing climatic factors. Therefore, plants need to regulate and adjust their cellular metabolism to optimize resource allocation between growth, storage, or production of defense compounds.

Plants have evolved a multi-layered and complex network of defense responses, including pre-formed physical barriers (e.g. bark and cuticle) and inducible perception systems, both at the cell surface and intracellularly, with the ability to discriminate between "self' and "non-self', "damaged-self' or "modified-self', which is the basis of immunity and evolutionary conserved. Major triggers of plant immunity are so-called microbe or pathogen-associated molecular patterns (MAMPs/PAMPs) or endogenous danger-associated patterns (DAMPs) that induce *de novo* production of anti-microbial defense proteins and metabolites, including phenolics, terpenes, alkaloids, and non-protein amino acids.

PAMP/MAMP/DAMP-triggered immunity protects plants against the majority of pathogens and reflects basal resistance PAMP/MAMPs/DAMPs are derivatives from major structural polysaccharides of the bacterial, fungal, or plant cell wall, for example lipopolysaccharides (LPS) from Gram- bacteria and peptidoglycan (PGN) from Gram+ bacteria, chitin and glucans from fungi, or pectin from plants. Furthermore, several proteins of fungal and bacterial origin trigger an immune response in plant cells. The most prominent examples are the bacterial flagellin (the major constituent of the bacterial flagellum), elongation factor thermo unstable (EF-Tu), and cold shock (CSP) proteins.

The present invention overcomes the shortcomings in the art by providing novel methods for increasing resistance to abiotic and biotic stresses.

### BRIEF DESCRIPTION OF THE FIGURES

The accompanying figures, which are incorporated herein and form a part of the specification, illustrate some, but not the only or exclusive, example embodiments and/or features. It is intended that the embodiments and figures disclosed herein are to be considered illustrative rather than limiting.
**Fig. 1** shows photographs of strawberries and onions harvested after treatment with a fungal mycelia extract (PRBT) (upper panel) versus control (untreated) strawberries and onion (lower panel).
**Fig. 2** shows photographs of soybean plants inoculated with *Sclerotinia sclerotiorum* and treated with PRBT (right panel) compared to *Sclerotinia sclerotiorum* inoculated control plants (middle panel) and mock inoculated control treated plants (left panel).
**Fig. 3** shows photographs of zucchini plants infected with tomato leaf curl New Dehli virus treated with PRBT (panel B) compared to control (untreated) plants (panel A). Inmunodetection membranes of 34 zucchini plants treated with PRBT (panel D) compared to control (untreated) plants (panel C) are shown where infected plants with tomato leaf curl New Dehli virus are highlighted with a solid line rectangle with tissue prints of the stem (left) and leaf (right) while negative (left) and positive controls (right) are indicated with a discontinuous rectangle.

### SUMMARY

The following embodiments and aspects thereof are described and illustrated in conjunction with products and methods, which are meant to be exemplary and illustrative, not limiting in scope. In some embodiments, one or more of the above-described problems have been reduced or eliminated, while other embodiments are directed to other improvements.

The present disclosure has applications in the agronomic sector. One embodiment discloses methods and compositions for increasing a growth characteristic of a plant comprising applying a composition comprising a fungal mycelia extract comprising piperidine and/or an analogue thereof (e.g., 6-oxopiperidine-2-carboxylic acid), a salt thereof, or any combination thereof (herein referred to as "PRBT") to a plant or part thereof. In some embodiments, a fungal mycelia extract comprises 6-oxopiperidine-2-carboxylic acid and/or an analogue and/or a salt thereof (e.g., 6-oxopiperidine-2-carboxylate).

An additional embodiment discloses increasing nutrient use efficiency of a plant comprising applying a composition comprising a fungal mycelia extract comprising piperidine and/or an analogue thereof (e.g., 6-oxopiperidine-2-carboxylic acid), a salt thereof, or any combination thereof to a plant or part thereof. In some embodiments, a fungal mycelia extract comprises 6-oxopiperidine-2-carboxylic acid and/or an analogue and/or a salt thereof (e.g., 6-oxopiperidine-2-carboxylate).

An additional embodiment discloses increasing biotic and/or abiotic stress tolerance or resistance in a plant or part thereof comprising applying a fungal mycelia extract comprising piperidine and/or an analogue thereof (e.g., 6-oxopiperidine-2-carboxylic acid), a salt thereof, or any combination thereof to a plant or part thereof. In some embodiments, a fungal mycelia extract comprises 6-oxopiperidine-2-carboxylic acid and/or an analogue and/or a salt thereof (e.g., 6-oxopiperidine-2-carboxylate).

In some embodiments, the fungal mycelial extract may further comprise peptides, proteins, carbohydrates and/or sugars. In some embodiments, a composition of the invention may further comprise, a surfactant, a humectant, an adjuvant, an antioxidant, a preservative, a plant macronutrient, a plant micronutrient, a plant growth regulator, a pesticide, a fungicide, an antiviral, an anti-bacterial, and/or an herbicide.

These and other aspects of the invention are set forth in more detail in the description of the invention below.

### DETAILED DESCRIPTION

The present invention now will be described hereinafter with reference to the accompanying drawings and examples, in which embodiments of the invention are shown. This description is not intended to be a detailed catalog of all the different ways in which the invention may be implemented, or all the features that may be added to the instant invention. For example, features illustrated with respect to one embodiment may be incorporated into other embodiments, and features illustrated with respect to a particular embodiment may be deleted from that embodiment. Thus, the invention contemplates that in some embodiments of the invention, any feature or combination of features set forth herein can be excluded or omitted. In addition, numerous variations and additions to the various embodiments suggested herein will be apparent to those skilled in the art in light of the instant disclosure, which do not depart from the instant invention. Hence, the following descriptions are intended to illustrate some particular embodiments of the invention, and not to exhaustively specify all permutations, combinations and variations thereof.

Unless otherwise defined, all technical and scientific terms used herein have the same meaning as commonly understood by one of ordinary skill in the art to which this invention belongs. The terminology used in the description of the invention herein is for the purpose of describing particular embodiments only and is not intended to be limiting of the invention.

All publications, patent applications, patents and other references cited herein are incorporated by reference in their entireties for the teachings relevant to the sentence and/or paragraph in which the reference is presented.

Unless the context indicates otherwise, it is specifically intended that the various features of the invention described herein can be used in any combination. Moreover, the present invention also contemplates that in some embodiments of the invention, any feature or combination of features set forth herein can be excluded or omitted. To illustrate, if the specification states that a composition comprises components A, B and C, it is specifically intended that any of A, B or C, or a combination thereof, can be omitted and disclaimed singularly or in any combination.

The terms "comprising," "having," "including," and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. For example, if the range 10-15 is disclosed, then 11, 12, 13, and 14 are also disclosed. All methods described herein can be performed in any suitable order unless otherwise indicated herein or otherwise clearly contradicted by context. The use of any and all examples, or exemplary language (e.g., "such as") provided herein, is intended merely to better illuminate the disclosure and does not pose a limitation on the scope of the disclosure unless otherwise claimed. No language in the specification should be construed as indicating any non-claimed element as essential to the practice of the disclosure.

As used in the description of the invention and the appended claims, the singular forms "a," "an" and "the" are intended to include the plural forms as well, unless the context clearly indicates otherwise.

Also as used herein, "and/or" refers to and encompasses any and all possible combinations of one or more of the associated listed items, as well as the lack of combinations when interpreted in the alternative ("or").

The term "about," as used herein when referring to a measurable value such as an amount or concentration and the like, is meant to encompass variations of ± 10%, ± 5%, ± 1%, ± 0.5%, or even ± 0.1% of the specified value as well as the specified value. For example, "about X" where X is the measurable value, is meant to include X as well as variations of ± 10%, ± 5%, ± 1%, ± 0.5%, or even ± 0.1% of X. A range provided herein for a measureable value may include any other range and/or individual value therein.

As used herein, phrases such as "between X and Y" and "between about X and Y" should be interpreted to include X and Y. As used herein, phrases such as "between about X and Y" mean "between about X and about Y" and phrases such as "from about X to Y" mean "from about X to about Y."

The term "comprise," "comprises" and "comprising" as used herein, specify the presence of the stated features, integers, steps, operations, elements, and/or components, but do not preclude the presence or addition of one or more other features, integers, steps, operations, elements, components, and/or groups thereof.

As used herein, the transitional phrase "consisting essentially of" means that the scope of a claim is to be interpreted to encompass the specified materials or steps recited in the claim and those that do not materially affect the basic and novel characteristic(s) of the claimed invention. Thus, the term "consisting essentially of" when used in a claim of this invention is not intended to be interpreted to be equivalent to "comprising."

As used herein, the terms "increase," "increasing," "increased," "enhance," "enhanced," "enhancing," and "enhancement" (and grammatical variations thereof) describe an elevation of at least about 25%, 50%, 75%, 100%, 150%, 200%, 300%, 400%, 500% or more as compared to a control.

As used herein, the terms "reduce," "reduced," "reducing," "reduction," "diminish," and "decrease" (and grammatical variations thereof), describe, for example, a decrease of at least about 5%, 10%, 15%, 20%, 25%, 35%, 50%, 75%, 80%, 85%, 90%, 95%, 97%, 98%, 99%, or 100% as compared to a control. In particular embodiments, the reduction can result in no or essentially no (*i.e.,* an insignificant amount, *e.g.,* less than about 10% or even 5%) detectable activity or amount.

As used herein, "growth characteristic" refers to any plant trait associated with growth, for example, biomass, yield, inflorescence size/weight, fruit yield, fruit quality, fruit size, seed production, foliar tissue weight, nodulation number, nodulation mass, nodulation activity, number of seed heads, number of tillers, number of flowers, number of tubers, tuber mass, bulb mass, number of seeds, total seed mass, rate of leaf, emergence, rate of tiller emergence, rate of seedling emergence or any combination thereof. Thus, in some aspects, an increased growth characteristic may be increased fruit production, increased inflorescence production, increased fruit quality, and/or increased biomass as compared to a control plant or part thereof to which the compositions of the invention have not been applied.

As used herein, "nutrient use efficiency" refers to a plant's ability to utilize available nutrients. In some embodiments, "nutrient use efficiency may be defined in terms of total nutrient uptake (nutrient concentration in plant tissue × total biomass) and/or yield per unit of nutrient applied.

The term "abiotic stress" as used herein refers to outside, nonliving, factors which can cause harmful effects to plants. Thus, as used herein, abiotic stress includes, but is not limited to, cold temperature that results in freezing, chilling or cold temperature, heat or high temperatures, drought, high light intensity, low light intensity, salinity, flooding (excess water/water-logging), ozone, and/or combinations thereof. Parameters for the abiotic stress factors are species specific and even variety specific and therefore vary widely according to the species/variety exposed to the abiotic stress. Thus, while one species may be severely impacted by a high temperature of 23°C, another species may not be impacted until at least 30°C, and the like. Temperatures above 30°C result in dramatic reductions in the yields of most important crops. This is due to reductions in photosynthesis that begin at approximately 20-25°C, and the increased carbohydrate demands of crops growing at higher temperatures. The critical temperatures are not absolute, but vary depending upon such factors as the acclimatization of the crop to prevailing environmental conditions. In addition, because most crops are exposed to multiple abiotic stresses at one time, the interaction between the stresses affects the response of the plant. Thus, the particular parameters for high/low temperature, light intensity, drought and the like, which impact crop productivity will vary with species, variety, degree of acclimatization and the exposure to a combination of environmental conditions.

The inventors of the present invention have discovered that treating plants or parts thereof with a composition comprising a fungal mycelia extract comprising piperidine and/or an analogue thereof (e.g., 6-oxopiperidine-2-carboxylic acid), a salt thereof (e.g., 6-oxopiperidine-2-carboxylate), or any combination thereof (also referred to herein as "PBRT") can increase a growth characteristic, nutrient use efficiency, and/or abiotic and/or biotic stress tolerance/resistance of the plant or part thereof. Accordingly, in some embodiments, the present invention provides a composition for increasing a growth characteristic of a plant or part thereof, for increasing nutrient use efficiency of a plant or part thereof, and/or for increasing abiotic stress and/or biotic stress tolerance of a plant or part thereof, the composition comprising an effective amount of a fungal mycelia extract comprising piperidine and/or an analogue thereof (e.g., 6-oxopiperidine-2-carboxylic acid), a salt thereof (e.g., 6-oxopiperidine-2-carboxylate), or any combination thereof. In some embodiments, the invention provides a composition comprising a fungal mycelia extract comprising piperidine and/or an analogue thereof (e.g., 6-oxopiperidine-2-carboxylic acid), a salt thereof (e.g., 6-oxopiperidine-2-carboxylate), or any combination thereof. Thus, in some embodiments, a fungal mycelia extract comprises 6-oxopiperidine-2-carboxylic acid and/or an analogue and/or a salt thereof (e.g., 6-oxopiperidine-2-carboxylate).

In some embodiments, a composition may comprise an amount of piperidine and/or an analogue thereof (e.g., 6-oxopiperidine-2-carboxylic acid), a salt thereof (e.g., 6-oxopiperidine-2-carboxylate) in a range from about 0.1 grams (g) per liter (L) to about 50 g/L of the composition. Thus, in some embodiments, a composition of the invention may comprise piperidine and/or an analogue thereof, or salt thereof in a range from about 0.1 g/L to about 1 g/L, about 0.1 g/L to about 5 g/L, about 0.1 g/L to about 10 g/L, about 0.1 g/L to about 15 g/L, about 0.1 g/L to about 20 g/L, about 0.1 g/L to about 30 g/L, about 0.1 g/L to about 40 g/L, about 0.5 g/L to about 1 g/L, about 0.5 g/L to about 5 g/L, about 0.5 g/L to about 10 g/L, about 0.5 g/L to about 20 g/L, about 0.5 g/L to about 30 g/L, about 0.5 g/L to about 40 g/L, about 0.5 g/L to about 50 g/L, about 1 g/L to about 5 g/L, about 1 g/L to about 10 g/L, about 1 g/L to about 15 g/L, about 1 g/L to about 20 g/L, about 1 g/L to about 30 g/L, about 1 g/L to about 40 g/L, about 1 g/L to about 50 g/L, about 5 g/L to about 10 g/L, about 5 g/L to about 15 g/L, about 5 g/L to about 20 g/L, about 5 g/L to about 30 g/L, about 5 g/L to about 40 g/L, 1, about 5 g/L to about 50 g/L, about 10 g/L to about 15 g/L, about 10 g/L to about 20 g/L, about 10 g/L to about 30 g/L, about 10 g/L to about 40 g/L, about 10 g/L to about 50 g/L, , about 15 g/L to about 20 g/L, about 15 g/L to about 30 g/L, about 15 g/L to about 40 g/L, about 15 g/L to about 50 g/L, about 20 g/L to about 30 g/L, or about 20 g/L to about 40 g/L, about 20 g/L to about 50 g/L, about 30 g/L to about 40 g/L, about 30 g/L to about 50 g/L, or about 40 g/L to about 50 g/L of the composition, or any value or range therein. In some embodiments, a composition of the invention may comprise piperidine and/or analogue thereof, or salt thereof in an amount of about 0.01, 0.05, 0.1, 0.5, 1, 1.5, 2, 2.5, 3, 3.5, 4, 4.5, 5, 5.5, 6, 6.5, 7, 7.5, 8, 8.5, 9, 9.5, 10, 10.5, 11, 11.5, 12, 12.5, 13, 13.5, 14, 14.5, 15, 15.5, 16, 16.5, 17, 17.5, 18, 18.5, 19, 19.5, 20, 21, 22, 23, 24, 25, 26, 27, 28, 29, 30, 31, 32, 33, 34, 35, 40, 45, or 50 g per liter of the composition, or any range or value therein. In some embodiments, the amount of piperidine and/or analogue thereof, or salt thereof in a composition of the invention may be in a range of about 0.1 g/L to about 5 g/L (e.g., about 0.01, 0.05, 0.1, 0.5, 1, 1.5, 2, 2.5, 3, 3.5, 4, 4.5, 5 g per liter of the composition, or any range or value therein).

In some embodiments, a composition of the invention may comprise a fungal mycelia extract (e.g., "PBRT") in an amount from about 0.01% to about 100% w/w of the composition. Thus, in some embodiments, a composition of the invention may comprise a fungal mycelia extract in an amount from about 0.01% to about 0.1%, about 0.01% to about 1%, about 0.01% to about 3%, about 0.01% to about 5%, about 0.01% to about 10%, about 0.01% to about 15%, about 0.01% to about 20%, about 0.01% to about 25%, about 0.01% to about 30%, about 0.01% to about 35%, about 0.01% to about 40%, about 0.01% to about 45%, about 0.01% to about 50%, about 0.01% to about 60%, about 0.01% to about 70%, about 0.01% to about 80%, about 0.01% to about 90%, about 0.01% to about 95%, 0.1% to about 1%, 0.1% to about 3%, 0.1% to about 5%, about 0.1% to about 10%, about 0.1% to about 15%, about 0.1% to about 20%, about 0.1% to about 25%, about 0.1% to about 30%, about 0.1% to about 35%, about 0.1% to about 40%, about 0.1% to about 45%, about 0.1% to about 50%, about 0.1% to about 60%, about 0.1% to about 70%, about 0.1% to about 80%, about 0.1% to about 90%, about 0.1% to about 100%, about 0.5% to about 1%, about 0.5% to about 3%, about 0.5% to about 5%, about 0.5 to about 10%, about 0.5 to about 15%, about 0.5% to about 20%, about 0.5% to about 25%, about 0.5% to about 30%, about 0.5% to about 35%, about 0.5% to about 40%, about 0.5% to about 45%, about 0.5% to about 50%, about 0.5% to about 60%, about 0.5% to about 70%, about 0.5% to about 80%, about 0.5% to about 90%, about 0.5% to about 100%, about 1% to about 3%, about 1% to about 5%, about 1% to about 10%, about 1% to about 15%, about 1% to about 20%, about 1% to about 25%, about 1% to about 30%, about 1% to about 35%, about 1% to about 40%, about 1% to about 45%, about 1% to about 50%, about 1% to about 60%, about 1% to about 70%, about 1% to about 80%, about 1% to about 90%, about 1% to about 100%, about 5% to about 10%, about 5% to about 15%, about 5% to about 20%, about 5% to about 25%, about 5% to about 30%, about 5% to about 35%, about 5% to about 40%, about 5% to about 45%, about 5% to about 50%, about 5% to about 60%, about 5% to about 70%, about 5% to about 80%, about 5% to about 90%, about 5% to about 100%, about 10% to about 15%, about 10% to about 20%, about 10% to about 25%, about 10% to about 30%, about 10% to about 35%, about 10% to about 40%, about 10% to about 45%, about 10% to about 50%, about 10% to about 60%, about 10% to about 70%, about 10% to about 80%, about 10% to about 90%, about 10% to about 100%, about 15% to about 20%, about 15% to about 25%, about 15% to about 30%, about 10% to about 35%, about 15% to about 40%, about 10% to about 45%, about 15% to about 50%, about 15% to about 60%, about 15% to about 70%, about 15% to about 80%, about 15% to about 90%, about 15% to about 100%, about 20% to about 25%, about 20% to about 30%, about 20% to about 35%, about 20% to about 40%, about 20% to about 45%, about 20% to about 50%, about 20% to about 60%, about 20% to about 70%, about 20% to about 80%, about 20% to about 90%, about 20% to about 100%, about 25% to about 30%, about 25% to about 35%, about 25% to about 40%, about 25% to about 45%, about 25% to about 50%, about 25% to about 60%, about 25% to about 70%, about 25% to about 80%, about 25% to about 90%, about 25% to about 100%, about 30% to about 35%, about 30% to about 40%, about 30% to about 45%, about 30% to about 50%, about 30% to about 60%, about 30% to about 70%, about 30% to about 80%, about 30% to about 90%, about 30% to about 100%, about 35% to about 40%, about 35% to about 45%, about 35% to about 50%, about 35% to about 60%, about 35% to about 70%, about 35% to about 80%, about 35% to about 90%, about 35% to about 100%, about 40% to about 45%, about 40% to about 50%, about 40% to about 60%, about 40% to about 70%, about 40% to about 80%, about 40% to about 90%, about 40% to about 100%, about 50% to about 60%, about 50% to about 70%, about 50% to about 75%, about 50% to about 95%, about 60% to about 80%, about 60% to about 90%, about 60% to about 95%, about 60% to about 100% , about 75% to about 80%, about 75% to about 90%, about 75% to about 100%, about 80% to about 95%, about 80% to about 100%, about 90% to about 95%,or about 90% to about 100% w/w of the composition, or any value or range therein. In some embodiments, a composition of the invention may comprise a fungal mycelia extract in an amount of about 0.01%, 0.05%, 0.1%, 0.5%, 1%, 1.5%, 2%, 2.5%, 3, 3.5%, 4%, 4.5%, 5%, 5.5%, 6%, 6.5%, 7%, 7.5%, 8%, 8.5%, 9%, 9.5%, 10%, 11%, 12%, 13%, 14%, 15%, 16%, 17, 5, 18%, 19%, 20%, 21%, 22%, 23%, 24%, 25%, 26%, 27%, 28%, 29%, 30%, 35%, 40%, 45%, or 50%, 55%, 60%, 65%, 70%, 75%, 80%, 85%, 90%, 95%, or 100% w/w of the composition, or any range or value therein.

In some embodiments, an effective amount of a fungal mycelia extract is an amount sufficient to increase a growth characteristic of a plant or part thereof, increase nutrient use efficiency in a plant or part thereof, and/or increase abiotic stress and/or biotic stress tolerance/resistance of a plant or part thereof. In some embodiments, an effective amount of a fungal mycelia extract in a composition may be from about 0.005 g per liter to about 150 g per liter of the composition. In some embodiments, an effective amount of a fungal mycelia extract in a composition may be from about 0.005 g/L to about 1 g/L, about 0.005 g/L to about 5 g/L, about 0.005 g/L to about 10 g/L, about 0.005 g/L to about 15 g/L, about 0.005 g/L to about 20 g/L, about 0.005 g/L to about 30 g/L, about 0.01 g/L to about 1 g/L, about 0.01 g/L to about 5 g/L, about 0.01 g/L to about 10 g/L, about 0.01 g/L to about 15 g/L, about 0.01 g/L to about 20 g/L, about 0.01 g/L to about 30 g/L, about 0.01 g/L to about 35 g/L, about 0.05 g/L to about 1 g/L, about 0.05 g/L to about 5 g/L, about 0.05 g/L to about 10 g/L, about 0.05 g/L to about 15 g/L, about 0.05 g/L to about 20 g/L, about 0.05 g/L to about 30 g/L, about 0.05 g/L to about 35 g/L, about 0.1 g/L to about 1 g/L, about 0.1 g/L to about 5 g/L, about 0.1 g/L to about 10 g/L, about 0.1 g/L to about 15 g/L, about 0.1 g/L to about 20 g/L, about 0.1 g/L to about 30 g/L, about 0.1 g/L to about 35 g/L, about 0.5 g/L to about 1 g/L, about 0.5 g/L to about 5 g/L, about 0.5 g/L to about 10 g/L, about 0.5 g/L to about 15 g/L, about 0.5 g/L to about 20 g/L, about 0.5 g/L to about 30 g/L, about 0.5 g/L to about 35 g/L, about 1 g/L to about 5 g/L, about 1 g/L to about 10 g/L, about 1 g/L to about 15 g/L, about 1 g/L to about 20 g/L, about 1 g/L to about 30 g/L, about 1 g/L to about 35 g/L, about 1 g/L to about 40 g/L, about 1 g/L to about 50 g/L, about 1 g/L to about 75 g/L, about 1 g/L to about 100 g/L, about 1 g/L to about 125 g/L, about 1 g/L to about 150 g/L, about 5 g/L to about 10 g/L, about 5 g/L to about 15 g/L, about 5 g/L to about 20 g/L, about 5 g/L to about 30 g/L, about 5 g/L to about 35 g/L, about 5 g/L to about 40 g/L, about 5 g/L to about 50 g/L, about 5 g/L to about 75 g/L, about 5 g/L to about 100 g/L, about 5 g/L to about 125 g/L, about 5 g/L to about 150 g/L, about 10 g/L to about 15 g/L, about 10 g/L to about 20 g/L, about 10 g/L to about 30 g/L, about 10 g/L to about 40 g/L, about 10 g/L to about 50 g/L, about 10 g/L to about 75 g/L, about 10 g/L to about 100 g/L, about 10 g/L to about 125 g/L, about 10 g/L to about 150 g/L, about 15 g/L to about 20 g/L, about 15 g/L to about 30 g/L, about 15 g/L to about 40 g/L, about 15 g/L to about 50 g/L, about 15 g/L to about 75 g/L, about 15 g/L to about 100 g/L, about 15 g/L to about 125 g/L, about 15 g/L to about 150 g/L, about 20 g/L to about 25 g/L, about 20 g/L to about 30 g/L, or about 20 g/L to about 40 g/L, about 20 g/L to about 50 g/L, about 20 g/L to about 75 g/L, about 20 g/L to about 100 g/L, about 20 g/L to about 125 g/L, about 20 g/L to about 150 g/L, about 30 g/L to about 60 g/L, about 30 g/L to about 75 g/L, about 30 g/L to about 90 g/L, about 30 g/L to about 100 g/L, about 30 g/L to about 125 g/L, about 30 g/L to about 150 g/L, about 40 g/L to about 60 g/L, about 40 g/L to about 75 g/L, about 40 g/L to about 90 g/L, about 40 g/L to about 100 g/L, about 40 g/L to about 125 g/L, about 40 g/L to about 150 g/L, about 50 g/L to about 75 g/L, about 50 g/L to about 90 g/L, about 50 g/L to about 100 g/L, about 50 g/L to about 125 g/L, about 50 g/L to about 150 g/L, about 60 g/L to about 75 g/L, about 60 g/L to about 90 g/L, about 60 g/L to about 100 g/L, about 60 g/L to about 125 g/L, about 60 g/L to about 150 g/L, about 75 g/L to about 90 g/L, about 75 g/L to about 100 g/L, about 75 g/L to about 125 g/L, about 75 g/L to about 150 g/L, about 95 g/L to about 110 g/L, about 95 g/L to about 120 g/L, about 95 g/L to about 130 g/L, about 95 g/L to about 150 g/L, about 100 g/L to about 120 g/L, about 100 g/L to about 130 g/L, about 100 g/L to about 140 g/L, about 100 g/L to about 150 g/L, about 120 g/L to about 150 g/L, about 135 g/L to about 150 g/L, or about 140 g/L to about 150 g/L of the composition, or any value or range therein. Thus, in some embodiments, an effective amount of a fungal mycelia extract in a composition may be about 0.005, 0.01, 0.05, 0.1, 0.5, 1, 1.5, 2, 2.5, 3, 3.5, 4, 4.5, 5, 5.5, 6, 6.5, 7, 7.5, 8, 8.5, 9, 9.5, 10, 10.5, 11, 11.5, 12, 12.5, 13, 13.5, 14, 14.5, 15, 15.5, 16, 16.5, 17, 17.5, 18, 18.5, 19, 19.5, 20, 21, 22, 23, 24, 25, 26, 27, 28, 29, 30, 31, 32, 33, 34, 35, 40, 45, 50, 55, 60, 65, 70, 75, 80, 85, 90, 95, 100, 105, 110, 115, 120, 125, 130, 135, 140, 145, or 150 g per liter of the composition, or any range or value therein).

An extract of the invention may comprise an analogue and/or salt of piperidine (e.g., - oxopiperidine-2-carboxylic acid, 6-oxopiperidine-2-carboxylate). As used herein an "analogue" of a respective compound may include, but is not limited to, a tautomer and/or an isomer (e.g., a stereoisomer and/or a structural isomer) of the respective compound. An "analogue" of a respective compound may also include a compound that has a similar or the same core structure (e.g., a core structure comprising piperidine) as the respective compound and optionally one or more substituent(s) that may be different than a substituent of the respective compound and/or in a different position compared to a substituent of the respective compound.

In some embodiments, an analogue of a compound of the present invention (e.g., piperidine, 6-oxopiperidine-2-carboxylic acid, and/or 6-oxopiperidine-2-carboxylate) may have a structure represented by Formula I: 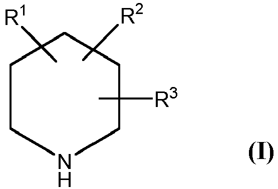 wherein
R¹ is selected from the group consisting of H and -COOH;
R² is selected from the group consisting of H, -OH, =O, -NH₂, and C₁-C₆ alkyl; and
R³ is selected from the group consisting of H, -OH, =O, -NH₂, and C₁-C₆ alkyl.

In some embodiments, in a compound of Formula I, R¹ is -COOH; R² is selected from the group consisting of H, -OH, =O, and NH₂; and R³ is selected from the group consisting of H, -NH₂, and C₁-C₄ alkyl.

The structure for 6-oxopiperidine-2-carboxylic acid is as follows. 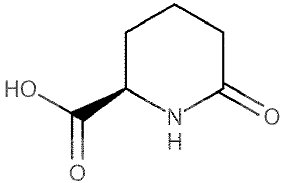 When 6-oxopiperidine-2-carboxylic acid is in the form of a salt, then it is referred to herein as 6-oxopiperidine-2-carboxylate.

An IUPAC name for 6-oxopiperidine-2-carboxylate includes 6-hydroxy-2,3,4,5-tetrahydropyridine-2-carboxylic acid. 6-oxopiperidine-2-carboxylate is also known as 6-oxopiperidine-2-carboxylic acid, adipo-2,6-Lactam, cyclic alpha-aminoadipic acid, 6-oxopiperidine-2-carboxylate, cyclic α-aminoadipate, cyclic α-aminoadipic acid, cyclic alpha-aminoadipate, cyclic α-aminoadipate, cyclic α-aminoadipic acid, or 6-oxo-pipecolinic acid.

An extract of the invention may comprise an analogue and/or salt of piperidine (e.g., - oxopiperidine-2-carboxylic acid, 6-oxopiperidine-2-carboxylate).

As used herein, an analogue of piperidine may include, but is not limited to, 6-oxopiperidine-2-carboxylic acid, 6-hydroxypiperidine-2-carboxylic acid; 6-oxopiperidine-3-carboxylic acid; 6-hydroxypiperidine-3-carboxylic acid; 3-hydroxypiperidine-2-carboxylic acid; 4-hydroxypiperidine-2-carboxylic acid; 5-oxopiperidine-2-carboxylic acid; 5-hydroxypiperidine-2-carboxylic acid; 5-oxopiperidine-3-carboxylic acid; 5-hydroxypiperidine-3-carboxylic acid; 4-oxopiperidine-2-carboxylic acid; 4-hydroxypiperidine-2-carboxylic acid; 4-oxopiperidine-3-carboxylic acid; 4-hydroxypiridine-3-carboxylic acid; 3-oxopiperidine-4-carboxylic-acid; 3-oxopiperidine-2 -carboxylic acid; 2-oxopiperidine-4 carboxylic acid; 2-oxopiperidine-3-carboxylic acid; 2-oxopiperidine-1-carboxylic acid; piperidine-4-carboxylic acid; piperidine-3-carboxylic acid; piperidine-2-carboxylic acid; piperidine-1-carboxylic acid; piperidine-4-carboxamide, piperidine-3-carboxamide; piperidine-2-carboxamide; 5-hydroxy-2-oxopiperidine-3-carboxylic acid; 5-hydroxy-2-oxopiperidine-4-carboxylic acid; 5-hydroxy-6-oxopiperidine-2-carboxylic acid; 5-hydroxy-4-oxopiperidine-2-carboxylic acid; 4-hydroxy-6-oxopiperidine-2-carboxylic acid; 4-hydroxy-6-oxopiperidine-3-carboxylic acid; 4-hydroxy-5-oxopiperidine-3-carboxylic acid; 3-hydroxy-6-oxopiperidine-2-carboxylic acid; 3-hydroxy-4-oxopiperidine-2-carboxylic acid; 3-hydroxy-2-oxopiperidine-4-carboxylic acid; 2-hydroxy-6-oxopiperidine-4-carboxylic acid; 5-amino-6-oxopiperidine-2-carboxylic acid; acid; 5-amino-6-oxopiperidine-3-carboxylic acid; 5-amino-3-oxopiperidine-2-carboxylic acid; 5- amino-2-oxopiperidine-3-carboxylic acid; 4-amino-6-oxopiperidine-2-carboxylic acid; 2-amino-6-oxopiperidine-4-carboxylic acid; methyl piperidine-4-carboxylic acid; methyl piperidine-3-carboxylic acid; methyl piperidine-2-carboxylic acid; methyl piperidine-1-carboxylic acid; methyl 6-oxopiperidine-3-carboxylic acid; methyl 6-oxopiperidine-2-carboxylic acid, methyl 4-oxopiperidine-3-carboxylic acid; methyl 2-oxopiperidine-3-carboxylic acid; 6-methyl-4-oxopiperidine-2-carboxylic acid; 6-methyl-2-oxopiperidine-3-carboxylic acid; 5-methyl-6-oxopiperidine- 2-carboxylic acid; 5-methyl-6-oxopiperidine-3-carboxylic acid; 5-methyl-2-oxopiperidine-1-carboxylic acid; 5-methyl-2-oxopiperidine-3-carboxylic acid; 5-methyl-2-oxopiperidine-4-carboxylic acid; 5-methyl-4-oxopiperidine-3-carboxylic acid; 4-methyl-6-oxopiperidine-2-carboxylic acid; 4-methyl-6-oxopiperidine-3-carboxylic acid; 4-methyl-2-oxopiperidine-3-carboxylic acid; 4-methyl-2-oxopiperidine-1-carboxylic acid; 3-methyl-6-oxopiperidine-2-carboxylic acid; 3-methyl-5-oxopiperidine-2-carboxylic acid; 2-methyl-6-oxopiperidine-4-carboxylic acid; 2-methyl-6-oxopiperidine-3-carboxylic acid; 1-methyl-6-oxopiperidine-2-carboxylic acid; 1-methyl-6-oxopiperidine-3-carboxylic acid; ethyl 4-oxopiperidine-3-carboxylic acid; ethyl 4-oxopiperidine-1-carboxylic acid; ethyl 3-oxopiperidine-2-carboxylic acid; ethyl 2-oxopiperidine-1-carboxylic acid. In some embodiments, an analogue of 6-oxopiperidine-2-carboxylate may be, for example, 6-hydroxypiperidine-2-carboxylic acid, 4-hydroxypiperidine-2-carboxylic acid, and/or 3-hydroxypiperidine-2-carboxylic acid.

A salt useful with this invention includes, but is not limited to, sodium, potassium, ammonium, copper, magnesium, calcium, zinc, molybdenum, iron, aluminium, lead, cadmium, chromium, nickel, mercury, and/or arsenic.

In some embodiments, a fungal mycelia extract may further comprise a peptide, a protein, a sugar and/or a carbohydrate. In some embodiments, a fungal mycelia extract may comprise peptides and/or proteins in an amount from about 0.1% to about 10% w/w of the extract. In some embodiments, a fungal mycelia extract may comprise peptides and/or proteins in an amount from about 0.1% to about 1%, about 0.1% to about 3%, about 0.1% to about 5%, about 0.1% to about 7%, about 0.5% to about 1%, about 0.5% to about 3%, about 0.5% to about 5%, about 0.5% to about 7%, about 0.5 to about 10%, about 1% to about 3%, about 1% to about 5%, about 1% to about 7%, about 1% to about 10%, about 3% to about 5%, about 3% to about 7%, about 3% to about 10%, about 5% to about 7%, about 5% to about 10%, or about 7% to about 10%, or any range or value therein of the composition. Thus, in some embodiments, a fungal mycelia extract may comprise peptides and/or proteins in an amount of about 0.1, 0.25, 0.5, 0.75, 1, 1.5, 2, 2.5, 3, 3.5, 4, 4.5, 5, 5.5, 6, 6.5, 7, 7.5, 8, 8.5, 9, 9.5, or 10% w/w or any range or value therein of the composition.

In some embodiments, a fungal mycelia extract may comprise sugars and/or carbohydrates in an amount from about 1% to about 35% w/w of the extract. In some embodiments, a fungal mycelia extract may comprise sugars and/or carbohydrates in an amount from about 1% to about 5%, about 1% to about 10%, about 1% to about 15%, about 1% to about 20%, about 1% to about 25%, about 1% to about 30%, about 5% to about 10%, about 5% to about 15%, about 5% to about 20%, about 5% to about 25%, about 5% to about 30%, about 5% to about 35%, about 10% to about 15%, about 10% to about 20%, about 10% to about 25%, about 10% to about 30%, about 10% to about 35%, about 15% to about 20%, about 15% to about 25%, about 15% to about 30%, about 15% to about 35%, about 20% to about 25%, about 20% to about 30%, about 20% to about 35%, about 25% to about 30%, about 25% to about 35%, or about 30% to about 35% w/w of the extract, or any value or range therein. Thus, in some embodiments, the fungal mycelia extract may comprise sugars and/or carbohydrates in an amount of about 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, or 35% w/w of the extract or any range or value therein.

In some embodiments, a sugar and/or carbohydrate that may be comprised in the fungal mycelia extract can include, but is not limited to glucose, mannose, galactose, glucan oligosaccharides, glucose-derived low branched polysaccharides, glycogen, mannan oligosaccharides, mannose-derived low branched polysaccharides, galactans, and/or galactomannans.

In some embodiments, a fungal mycelia extract may comprise piperidine and/or an analogue thereof, a salt thereof, and/or any combination thereof in an amount from about 1% to about 40% w/w of the extract. In some embodiments, a fungal mycelia extract may comprise piperidine and/or an analogue thereof, a salt thereof, and/or any combination thereof in an amount from about 1% to about 5%, about 1% to about 10%, about 1% to about 15%, about 1% to about 20%, about 1% to about 25%, about 1% to about 30%, about 1% to about 35%, about 5% to about 10%, about 5% to about 15%, about 5% to about 20%, about 5% to about 25%, about 5% to about 30%, about 5% to about 35%, about 5% to about 40%, about 10% to about 15%, about 10% to about 20%, about 10% to about 25%, about 10% to about 30%, about 10% to about 35%, about 10% to about 40%, about 15% to about 20%, about 15% to about 25%, about 15% to about 30%, about 15% to about 35%, about 15% to about 40%, about 20% to about 25%, about 20% to about 30%, about 20% to about 35%, about 20% to about 40%, about 25% to about 30%, about 25% to about 35%, about 25% to about 40%, about 30% to about 35%, about 30% to about 40%, about 35% to about 40% w/w of the extract, or any value or range therein. Thus, in some embodiments, a fungal mycelia extract may comprise piperidine and/or an analogue thereof, a salt thereof, and/or any combination thereof in an amount of about 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40% w/w of the extract or any range or value therein.

In some embodiments, a composition of the invention may further comprise additional components including, but not limited to, a surfactant, a humectant, an adjuvant, an antioxidant, a preservative, a plant macronutrient, a plant micronutrient, a plant growth regulator, a pesticide, a fungicide, an antiviral, an anti-bacterial, a herbicide, or any combination thereof.

Example surfactants can include, but are not limited to, alkali metal, alkaline earth metal and ammonium salts of ligno-sulfonic acid, naphthalenesulfonic acid, phenolsulfonic acid, dibutylnaphthalenesulfonic acid, alkylarylsulfonates, sodium dodecylsulfate, alkyl sulfates, alkylsulfonates, fatty alcohol sulfates, fatty acids and sulfated fatty alcohol glycol ethers, of sulfonated condensates naphthalene and naphthalene derivatives with formaldehyde, condensates of naphthalene or of naphthalenesulfonic acid with phenol and formaldehyde, polyoxyethylene octyl-phenyl ether, ethoxylated isooctylphenol, octylphenol, nonylphenol, alkylphenyl poly-glycol ethers, tributylphenyl polyglycol ether, tristearylphenyl polyglycol ether, alkylaryl polyether alcohols, alcohol and fatty alcohol/ethylene oxide condensates, ethoxylated castor oil, polyoxyethylene alkyl ethers, ethoxylated polyoxypropylene, lauryl alcohol, polyglycol ether acetal, sorbitol esters, lignin-sulfite waste liquors and/or methylcellulose.

In some embodiments, a surfactant may be present in a composition in an amount from about 10% to about 40% w/w of the composition. In some embodiments, a surfactant may be present in a composition in an amount from about 10% to about 15%, about 10% to about 20%, about 10% to about 25%, about 10% to about 30%, about 10% to about 35%, about 15% to about 20%, about 15% to about 25%, about 15% to about 30%, about 15% to about 35%, about 15% to about 40%, about 20% to about 25%, about 20% to about 30%, about 20% to about 35%, about 20% to about 40%, about 25% to about 30%, about 25% to about 35%, about 25% to about 40%, about 30% to about 35%, about 30% to about 40%, about 35% to about 40% w/w of the composition or any range or value therein). Thus, in some embodiments, the surfactant may be present in a composition in an amount of about 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40% w/w of the composition or any range or value therein.

Example humectants can include, but are not limited to, glycerol, sorbitol, xylitol, maltitol, glyceryl triacetate, sodium lactate, urea formaldehyde, propylene glycol, ethylene glycol and/or fatty acids.

An example antioxidant can include, but is not limited to, ascorbic acid, tocopherols, propyl gallate, tertiary butylhydroquinone, butylated hydroxyanisole, and/or butylated hydroxytoluene.

An example preservative can include, but is not limited to, sorbic acid, sodium sorbate, sorbates, benzoic acid, sodium benzoate, benzoates, hydroxybenzoate and derivatives, sulfur dioxide and sulphites, nitrite, nitrate, lactic acid, propionic acid and sodium propionate, tocopherol, plant extract, hops, salt, sugar, vinegar, alcohol (e.g. methanol and ethanol), diatomaceous earth and castor oil, citric acid, ascorbic acid, sodium ascorbate, phenol derivatives (butylated hydroxytoluene, butylated hydroxyanisole, BHA, BHT, TBHQ and propyl gallate), gallic acid, sodium gallate, sulfur dioxide, sulphites, tocopherols, and/or methylchloroisothiazolinone, 1,2-Benzisothiazolin-3-one (BIT), Hexahydro-1,3,5-tris-hydroxyethyl -s-triazine (HTHT), 5-chloro-2-methyl-2H-isothiazol-3-one (CMIT), 2-methyl-2H-isothiazol-3-one (MIT), Zinc pyrithione (ZPT), 2-Bromo-2-nitropropane-1,3-diol (Bronopol), Formaldehyde, 1,3-Dimethylol-5,5-dimethylhydantoin (DMDMH), 2,2-Dibromo-3-nitrilopropionamide (DBNPA), and/or Poly (hexamethylene biguanide) hydrochloride (PHMB).

In some embodiments, a preservative may be present in a composition in a range of about 0.001% to about 5% w/w or any range or value therein. In some embodiments, a composition may comprise a preservative in an amount ranging from about 0.001% to about 0.1%, about 0.001% to about 0.5%, about 0.001% to about 1%, about 0.001% to about 2%, about 0.001% to about 3%, about 0.001% to about 4%, about 0.01% to about 0.1%, about 0.01% to about 0.5%, about 0.01% to about 1%, about 0.01% to about 2%, about 0.01% to about 3%, about 0.01% to about 4%, about 0.01% to about 5%, about 0.05% to about 0.1%, about 0.05% to about 0.5%, about 0.05% to about 1%, about 0.05% to about 2%, about 0.05% to about 3%, about 0.05% to about 4%, about 0.05% to about 5%, about 0.1% to about 0.5%, about 0.1% to about 1%, about 0.1% to about 2%, about 0.1% to about 3%, about 0.1% to about 4%, about 0.1% to about 5%, about 0.5% to about 1%, about 0.5% to about 2%, about 0.5% to about 3%, about 0.5% to about 4%, about 0.5% to about 5%, about 1% to about 2%, about 1% to about 3%, about 1% to about 4%, about 1% to about 5%, about 2% to about 3%, about 2% to about 4%, about 4% to about 5%, about 3% to about 4%, about 3% to about 5%, about 4% to about 5% w/w of the composition, or any range or value therein. Thus, in some embodiments, the antifoaming agent may be present in the composition in an amount of about 0.001%, 0.002%, 0.003%, 0.004%, 0.005%, 0.006%, 0.007%, 0.008%, 0.009%, 0.01%, 0.02%, 0.03%, 0.04%, 0.05%, 0.06%, 0.07%, 0.08%, 0.09%, 0.1%, 0.2%, 0.3%, 0.4%, 0.5%, 0.6%, 0.7%, 0.8%, 0.9%, 1%, 1.1%, 1.2%, 1.3%, 1.4%, 1.5%, 1.6%, 1.7%, 1.8%, 1.9%, 2%, 2.5%, 3%, 3.5%, 4%, 4.5%, 5% w/w of the composition or any range or value therein.

Example plant macronutrients include, but are not limited to, nitrogen, potassium, calcium, magnesium, phosphorus, and/or sulfur.

Example plant micronutrients can include, but are not limited to, iron, manganese, boron, molybdenum, copper, zinc, chlorine, and/or cobalt.

Example plant growth regulators include, but are not limited to, auxin (including but not limited to naphthalene acetic acid (NAA) and/or indole-3-butyric acid (IBA) and/or indole-3-acetic acid (IAA, 3-IAA)), cytokinin, abscisic acid, gibberellin, ethylene, salicylic acid, jasmonic acid, brassinosteriod (e.g., brassinolide), or any combination thereof.

Example pesticides include, but are not limited to, malathion, parathion, methyl parathion, chlorpyrifos, diazinon, dichlorvos, phosmet, fenitrothion, tetrachlorvinphos, azamethiphos, fenvalerate, cyfluthrin, lambda-cyhalothrin, zeta-cypermethrin, permethrin, piperonyl butoxide, imidacloprid, acetamiprid, clothianidin, nitenpyram, nithiazine, thiacloprid, thiamethoxam, ryanodol, 9,21-didehydroryanodol, chlorantraniliprole, flubendiamide, and/or cyantraniliprole.

Example fungicides include, but are not limited to, prothioconazole trifloxystrobin, azoxystrobin, propiconazole, and/or pyraclostrobin.

Example anti-bacterials (bactericides) include, but are not limited to, methylisothiazolinone, chloromethylisothiazo linone, benzisothiazo linone, octylisothiazolinone, dichlorooctylisothiazolinone, and/or butylbenzisothiazolinone

Example herbicides can include, but are not limited to, glyphosate, 2,4-dichlorophenoxyacetic acid, atrazine, S-metolachlor, and/or 3,6-dichloro-2-methoxybenzoic acid.

In some embodiments, a composition of the invention may further comprise an antifoaming agent. Any antifoaming agent for use with agricultural and/or food products may be used. Example antifoaming agents include, but are not limited to, long chain unsaturated fatty acids including, but not limited to C12 to C14, C18:1 and C18:2 unsaturated fatty acids, and/or synthetic polysiloxanes (silicones) including, but not limited to, polydimethylsiloxane, and/or hydrophobic silica. In some embodiments, a composition of the invention may comprise an amount of antifoaming agent in a range from about 0.0001% to about 0.05% w/w of the composition or any range or value therein. Thus, in some embodiments, the antifoaming agent may be present in the composition in an amount of about 0.0001%, 0.0002%, 0.0003%, 0.0004%, 0.0005%, 0.0006%, 0.0007%, 0.0008%, 0.0009%, 0.001%, 0.002%, 0.003%, 0.004%, 0.005%, 0.006%, 0.007%, 0.008%, 0.009%, 0.01%, 0.02%, 0.03%, 0.04%, or 0.05% w/w of the composition or any range or value therein.

In some embodiments, a composition of the invention may further comprise a beneficial microbe. In some embodiments, the beneficial microbe may be *Bacillus subtilis, Pseudomonas* spp, *Azotobacter* spp, *Azospirillum* spp, *Rhizobium* spp, *Azorhizobium* spp, *Chaetomium* spp, *Streptomyces* spp. *Trichoderma* spp., and/or mycorrhizal fungi.

In some embodiments, a composition of the invention may be in the form of an aqueous solution, a non-aqueous solution, a suspension, a gel, a foam, a paste, a powder, a dust, a solid, and/or an emulsion.

**Example preparation of a fungal mycelial extract (PRBT)** (e.g., a fungal mycelia extract comprising piperidine and/or an analogue thereof, a salt thereof, or any combination thereof).

To prepare a mycelial extract (PRBT), about 150 g of dry mycelium from *Penicillium spp.* may be added to 1 liter (L) of water and stirred while heated at about 90 °C for about 3 hours. The mixture may be centrifuged and the resulting the supernatant may contain about 25 to about 30 g L⁻¹ of dry matter (upon removal of the water by evaporation or freeze-drying).

PRBT may be used directly in the methods of the present invention or PRBT may be formulated to comprise additional components. Thus, as an example, the supernatant obtained from the preparation of a fungal mycelial extract (as an example, see above) can be mixed with an anti-foaming agent to produce a mixture. When used, an anti-foaming agent may be present in a range from about 0.001 g L⁻¹ to about 0.5 g L⁻¹ of. In some embodiments, the amount of anti-foaming agent may be present in an about 0.0001% to about 0.05% w/w of the composition.

In some embodiments, a surfactant may be added to the supernatant (e.g., fungal mycelial extract) at a ratio of about 2:1.5 v/v about 2:0.5 v/v. As an example, the supernatant may be mixed in a proportion of 2:1 v/v with a surfactant. In some embodiments, a surfactant may be added to a composition of the invention in a range from about 10% to about 40% w/w of the composition.

In some embodiments, a biocide may be added to the supernatant. When included in a composition of the invention, a biocide may be present at about 0.5 g L⁻¹ to about 20 g L⁻¹.

A final mixture (e.g., a composition of the invention) may contain about 15 to about 20 g L⁻¹ of dry matter (of the fungal mycelial extract) when mixed in a proportion of 2:1 v/v with a surfactant.

A fungal extract (PBRT) of the invention may be concentrated using any method including, but not limited to, lyophilisation (e.g., freezing and drying down). The PRBT may be used directly, in a concentrated form, or prior to use, PRBT may be diluted to concentrations ranging from, for example, about 0.005 g L⁻¹ to about 20 g L⁻¹ or more.

In some embodiments, the supernatant may be mixed with a surfactant only (e.g., no antifoaming agent or biocide) in a proportion of about 2:1 v/w.

While the above example provides for a starting material of about 150 g of dry mycelium, as will be understood by those skilled in the art any amount of dry mycelium may be used, for example, from about 0.01 g to about 300 g.

While the above example provides for centrifugation to obtain the supernatant, it will be understood by those skilled in the art that other separation methods such as, e.g., filtration or decanting may be used.

While the above example used mycelium from *Penicillium spp.,* as will be understood by those skilled in the art other genera of fungi may be used.

While the above example provides for heating at about 90°C, as will be understood by those skilled in the art to prepare a fungal mycelia extract, the mycelia and water may be heated to a temperature from about 40°C to about 120°C. Thus, in some embodiments, the temperature for preparing a fungal mycelia extract may range from about 40°C to about 70°C, about 40°C to about 90°C, about 40°C to about 110°C, about 50°C to about 70°C, about 50°C to about 90°C, about 50°C to about 120°C, about 70°C to about 90°C, about 70°C to about 100°C, about 70°C to about 120°C, about 80°C to about 100°C, about 80°C to about 110°C, about 80°C to about 120°C, about 90°C to about 100°C, about 90°C to about 110°C, or about 90°C to about 120°C, or any range or value therein. In some embodiments, the temperature for preparing the mycelia extract may be about 40°C, 45°C, 50°C, 55°C, 60°C, 65°C, 70°C, 75°C, 80°C, 85°C, 90°C, 95°C, 100°C, 105°C, 110°C, 115°C, or 120°C or any range or value therein.

While the above example provides a 2:1 v/v supernatant (e.g., extract) to surfactant, as will be understood by those skilled in the art, additional ratios may be used, for example, about 2:1.5 v/v about 2:0.5 v/v.

While the above example provides for a biocide added at 2 g L⁻¹, as will be understood by those skilled in the art when a biocide is included in the composition of the invention, a biocide may be added in an amount, for example, ranging from about 0.5 g to about 2 g per liter (e.g., about 0.5, 0.6, 0.7, 0.8, 0.9, 1, 1.1, 1.2, 1.3, 1.4, 1.5, 1.6, 1.7, 1.8, 1.9, or 2 g per liter).

The above composition may further comprise micronutrients, such as, magnesium (Mg), boron (B), copper (Cu), iron (Fe), manganese (Mn), molybdenum (Mo) and zinc (Zn), fungicides, herbicides, insecticides, macronutrients, such as nitrogen (N) phosphorus (P) and potassium (K), and adjuvants.

A composition of the invention may be applied to plants or parts thereof for, for example, increasing a growth characteristic, increasing nutrient use efficiency, for increasing disease tolerance (biotic stress, e.g., tolerance to fungal, bacterial, and/or viral diseases) and/or for increasing abiotic stress tolerance. Thus, in some embodiments, the present invention provides a method for increasing a growth characteristic of a plant or part thereof, the method comprising applying a composition comprising an effective amount of a fungal mycelia extract to a plant or plant part thereof, wherein the extract comprises 6-oxopiperidine-2-carboxylate and/or piperidine, and or an analogue thereof, or a salt thereof, thereby increasing the growth characteristic of the plant or part thereof as compared to a control plant or part thereof (e.g., a plant or part thereof to which the composition or extract of the invention is has not been applied). In some embodiments, the method comprises applying a composition at least once (e.g., about 1, 2, 3, 4, 5, 6, 7, 8, 9, 10, 11, 12 or more times). In some embodiments, the method comprises applying the composition at least two times (e.g., about 2, 3, 4, 5, 6, 7, 8, 9, 10, 11, 12 or more times).

As used herein, a "prior application" refers to any application of a composition of the invention to a plant or plant part that is followed by another application (e.g., a subsequent application) of the composition.

In some embodiments, a method for increasing nutrient use efficiency of a plant or part thereof is provided, the method comprising applying a composition comprising an effective amount of a fungal mycelia extract to a plant or plant part thereof, wherein the extract comprises 6-oxopiperidine-2- carboxylate and/or piperidine, and or an analogue thereof, a salt thereof, or any combination thereof, thereby increasing nutrient use efficiency of the plant or part thereof as compared to a control plant or part thereof (e.g., a plant or part thereof to which the composition or extract of the invention is has not been applied). In some embodiments, the method comprises applying the composition at least once (e.g., about 1, 2, 3, 4, 5, 6, 7, 8, 9, 10, 11, 12 or more times). In some embodiments, the method comprises applying the composition at least two times (e.g., about 2, 3, 4, 5, 6, 7, 8, 9, 10, 11, 12 or more times).

In some embodiments, the present invention provides a method for increasing disease tolerance of a plant or part thereof, the method comprising applying a composition comprising an effective amount of a fungal mycelia extract to a plant or plant part thereof, wherein the extract comprises 6-oxopiperidine-2- carboxylate and/or piperidine, and or an analogue thereof, a salt thereof, or any combination thereof; thereby increasing the disease tolerance of the plant or part thereof as compared to a control plant or part thereof (e.g., a plant or part thereof to which the composition or extract of the invention is has not been applied). In some embodiments, the method comprises applying the composition at least once (e.g., about 1, 2, 3, 4, 5, 6, 7, 8, 9, 10, 11, 12 or more times). In some embodiments, the method comprises applying the composition at least two times (e.g., about 2, 3, 4, 5, 6, 7, 8, 9, 10, 11, 12 or more times). In some embodiments, the he method comprises applying the composition at least three times. In some embodiments, the method comprises applying the composition at least four times.

In some embodiments, when a composition of the invention is applied to a plant or plant part more than once, the range of time between treatments may vary. Thus, for example, a subsequent application (e.g., any application following a prior application; e.g., a second, third, fourth, fifth, sixth, seventh, eighth, ninth, tenth, eleventh, twelfth application and so on) of a composition of the invention may be any time from about 1 week to about six month after the prior application. Thus, for example, a subsequent application may be applied about 1, 2, 3, 4, 5, 6, 7, 8, 9, 10, 11, 12, 13, 14, 15, 16, 17, 18, 19, 20, 21, 22, 23, 24, 25, or 26 weeks after a prior application or about 0.2, 0.3, 0.4, 0.5, 0.6, 0.7, 0.8, 0.9, 1, 2, 3, 4, 5, 6 months after a prior application.

In some embodiments, applying a composition of the invention to a plant or part thereof may increase disease tolerance or resistance to a viral pathogen including, but not limited to, a virus from the virus family of *Caulimoviridae, Potyviridae, Sequiviridae, Rheoviridae, Capillovirus, Geminiviridae, Bromoviridae, Closteroviridae, Comoviridae Tombusviridae, Rhabdoviridae, Bunyaviridae, Partitiviridae, Carlavirus, Enamovirus, Furovirus, Hordeivirus, Idaeovirus, Luteovirus, Marafivirus, Potexvirus, Sobemovirus, Tenuivirus, Tobamovirus, Tobravirus, Trichovirus, Tymovirus* and/or *Umbravirus.*

In some embodiments, applying a composition of the invention to a plant or part thereof may increase resistance to a virus, including but not limited to, turnip mosaic virus, papaya ring spot virus, bud blight virus, bean pod mottle virus, lettuce mosaic virus, maize mosaic virus, cauliflower mosaic virus, tobacco mosaic virus, soybean mosaic virus, African cassava mosaic virus, tomato mosaic virus, pepino mosaic virus, zucchini yellow mosaic virus, plum pox virus, tomato bushy stunt virus, tomato spot wilt virus, tomato yellow leaf curl virus, rice ragged stunt virus, rice tungro bacilliform, virus, rice tungro spherical virus, rice yellow mottle virus, cucumber mosaic virus, brome mosaic virus, wheat yellow mosaic virus, barley yellow dwarf virus, sugarcane mosaic virus, beet yellows virus, lettuce yellows virus, maize dwarf mosaic virus, maize streak virus, peanut stunt virus, Citrus tristeza virus, potato leafroll virus, potato virus X, potato virus Y, sweet potato feathery mottle potyvirus, Melon necrotic spot virus, maize white line mosaic virus, maize chlorotic mottle virus, banana bunchy top virus, cacao swollen shoot virus, tomato leaf curl New Dehli virus, banana streak virus, and/or sweet potato sunken vein closterovirus.

In some embodiments, applying a composition of the invention to a plant or part thereof may increase resistance to a fungal pathogen including, but not limited to, a fungal pathogen from the family of Physodermataceae, Synchytriaceae, Olpidiaceae, Choanephoraceae, Gilbertellaceae, Mucoraceae, Dipodascaceae, Eremotheciaceae, Taphrinaceae, Botryosphaeriaceae, Capnodiaceae, Phaeosphaeriaceae, Leptosphaeriaceae ,Cucurbitariaceae, Didymellaceae Davidiellaceae, Mycosphaerellaceae, Schizothyriaceae, Dothideaceae, Dothioraceae, Lahmiaceae, Elsinoaceae, Lophiostomataceae, Pleosporaceae, Venturiaceae, Trichochomaceae, Erysiphaceae, Cyttariaceae, Hemiphacidiaceae, Hyaloscyphaceae, Phacidiaceae, Sclerotiniaceae, Ascodichaenaceae, Mediolariaceae, Rhytismataceae, Meliolaceae, Caloscyphaceae, Sarcosomataceae, Cryphonectriaceae, Diaporthaceae, Gnomoniaceae, Valsaceae, Glomerellaceae, Plectosphaerellaceae, Bionectriaceae, Clavicipitaceae, Hypocreaceae, Nectriaceae, Magnaporthaceae, Pyriculariaceae, Ceratocystideae, Ophiostomataceae, Phyllachoraceae, Chaetomiaceae, Amphisphaeriaceae, Diatrypaceae, Xylariaceae, Psathyrellaceae, Marasmiaceae, Mycenaceae, Schizophyllaceae, Typhulaceae,Thelephoraceae, Atheliaceae, Atheliaceae, Stereaceae, Echinodontiaceae, Corticiaceae, Ganodermataceae, Hymenochaetaceae, Cystofilobasidiaceae, Helicobasidiaceae, Helicobasidiaceae, Melampsoraceae, Phakopsoraceae, Pucciniaceae, Tilletiaceae, Entylomataceae, Ustilaginaceae, and/or Peronosporaceae.

In some embodiments, applying a composition of the invention to a plant or part thereof may increase resistance to a fungal pathogen including, but not limited to, *Physoderma alfalfa, Physoderma maydis, Synchytrium endobioticum, Olpidium brassicae, Choanephora cucurbitarum, Mucor circinelloides, Rhizopus stolonifera, Geotrichum candidum, Taphrina caerulescens, Taphrina deformans, Taphrina populina, Botryosphaeria dothidea, Diplodia mutila, Dothiorella sarmentorum, Macrophomina phaseolina, Phyllosticta ampelicida, Phyllosticta citricarpa, Stenocarpella maydis, Cladosporium alliicepae, Cladosporium cladosporioides, Acrodontium simplex, Cercospora spp., Cercospora apii, Cercospora beticola, Cercospora brassicicola, Cercospora kikuchii, Corynespora cassiicola, Cercospora zeae-maydis, Cercospora zeina, Dothistroma septosporum, Lecanosticta acicula, Mycocentrospora acerina, Passalora spp., Pseudocercospora fijiensis, Aureobasidium spp., Ophiosphaerella herpotricha, Parastagonospora nodorum, Diplodia tumefaciens, Alternaria alternate, Bipolaris maydis, Bipolaris oryzae, Bipolaris sacchari, Bipolaris victoriae, Curvularia spp., Leptosphaerulina trifolii, Venturia inaequalis, Aspergillus spp., Aspergillus flavus, Blumeria graminis, Erysiphe spp., Podosphaera leucotricha, Botrytis cinerea, Monilinia spp., Monilinia fructicola, Sclerotinia sclerotiorum, Amphilogia gyrosa, Cryphonectria parasitica, Diaporthe citri, Diaporthe helianthi, Diaporthe phaseolorum, Cytospora leucostoma, Colletotrichum spp., Colletotrichum coccodes, Colletotrichum gloeosporioides, Colletotrichum graminicola, Plectosphaerella cucumerina, Verticillium albo-atrum, Verticillium dahlia, Claviceps purpurea, Epichloe typhina, Trichoderma viride, Fusarium spp.* , *Fusarium oxysporum, Fusarium solani, Fusarium graminearum, Nectria cinnabarina, Neonectria spp., Gaeumannomyces graminis, Pyricularia grisea, Pyricularia oryzae, Ceratocystis spp., Thielaviopsis basicola, Ophiostoma ulmi, Phyllachora graminis, Cronartium spp., Uromyces graminicola, Tranzschelia spp., Tilletia spp., Ustilago spp., Ustilago maydis, Peronospora spp., Phytophthora spp., Pythium spp., Magnaporthe oryzae, Puccinia, Blumeria graminis, Exserohilum turcicum, Mycosphaerella graminicola, Melampsora lini, Phakopsora pachyrhizi,* and/or *Rhizoctonia solani.*

In some embodiments, applying a composition of the invention to a plant or part thereof may increase resistance to a bacterial pathogen including, but not limited to, a bacterial pathogen from the family of Enterobacteriaceae, Pseudomonadaceae, Rhizobiaceae, Microbacteriaceae, Xanthomonadaceae, Rhizobiaceae, Corynebacteriaceae, Acetobacteraceae, Comamonadaceae, Bacillaceae, Burkholderiaceae, Micrococcaceae, Ralstoniaceae, Xanthomonadaceae, Spiroplasmataceae, Sphingomonadaceae, Acholeplasmataceae, Corynebacteriaceae, and/or Streptomycetaceae. In some embodiments, applying a composition of the invention to a plant or part thereof may increase resistance to a bacterial pathogen including, but not limited to, a bacterial pathogen from the genus of *Erwinia* spp., *Pseudomonas* spp., *Xanthomonas* spp., *Agrobacterium* spp., *Rhizobium* spp., *Corynebacterium* spp., *Streptomyces* spp., *Pantoea* spp., *Serratia* spp., *Acetobacter* spp., *Acidovorax* spp., *Arthrobacter* spp., *Bacillus* spp., *Brenneria* spp., *Burkholderia* spp., *Clavibacter* spp., *Pectobacterium* spp., *Pantoea* spp., *Ralstonia* spp., *Xylella* spp., *Spiroplasma* spp., and *Phytoplasma* spp., *and*/*or Sphingomonas* spp.

In some embodiments, applying a composition of the invention to a plant or part thereof may increase resistance to a bacterial pathogen including, but not limited to, *Erwinia amylovora, E.a carotovora var. chrysanthemi. Pseudomonas tabaci, P. angulate, P. phaseolicola, P. lachrymans, P. pisi, P. fluorescens, P. glycinea, P. vesicatoria, P. savastanoi, P. syringae, P. solanacearum, Xanthamonas phaseoli, X. malvacearum, X. oryzae, X translucens, X. pruni, X. campestris, X. vasuclarum, Acidovorax avenae, Agrobacterium tumefaciens, A. rubi* (=*Rhizobium rubi), A. rhizogenes* (=*Rhizobium* rhizogenes) and *A. vitis* (=*Rhizobium vitis*), *Bacillus pumilus., Brenneria alni* (= *Erwinia alni*), *Clavibacter michiganensis, Pectobacterium carotovorum, Pantoea agglomerans, Ralstonia solanacearum, Corynebacterium insidiosum, C. sepedonicum, C. fascians,C. flacumfaciens, C. michiganense, Streptomyces scabies, S. ipomoeae, Pantoea agglomerans, Serratia marcescens, Streptomyces reticuliscabei, Acetobacter aceti, Spiroplasma citri Xylella fastidiosa, and*/or *Sphingomonas melonis.*

As used herein, "disease resistance" or "disease tolerance" are used interchangeably and refer to a decrease in disease symptoms and/or a decrease pathogen growth and reproduction of a plant or plant part. In some embodiments, the percent (%) increase in resistance/tolerance to disease as compared to a control may be in a range from about 0.1% to about 100%. In some embodiments, the percent increase in resistance/tolerance to disease may be an increase in a range from about 0.1% to about 10%, 0.1% to about 30%, about 0.1% to about 50%, about 0.1% to about 80%, about 0.1% to about 90%, about 0.1% to about 95%, about 1% to about 10%, about 1% to about 20%, about 1% to about 40%, about 1% to about 50%, about 1% to about 75%, about 1% to about 95%, about 1% to about 100%, about 10% to about 20%, about 10% to about 40%, about 10% to about 50%, about 10% to about 70%, about 10% to about 80%, about 10% to about 90%, about 10% to about 100%, about 20% to about 40%, about 20% to about 75%, about 20% to about 90%, about 20% to about 95%, about 20% to about 100%, about 25% to about 50%, about 50% to about 75%, about 50% to about 95%, about 50% to about 100%, about 75% to about 90%, about 75% to about 100%, about 90% to about 95%, about 90% to about 100% or any value or range therein, as compared to a control. In some embodiments, the % increase in resistance/tolerance to disease may be about 0.1%, 0.5%, 1%, 1.5%, 2%, 2.5%, 3, 3.5%, 4%, 4.5%, 5%, 5.5%, 6%, 6.5%, 7%, 7.5%, 8%, 8.5%, 9%, 9.5%, 10%, 11%, 12%, 13%, 14%, 15%, 16%, 17, 5, 18%, 19%, 20%, 21%, 22%, 23%, 24%, 25%, 26%, 27%, 28%, 29%, 30%, 35%, 40%, 45%, or 50%, 55%, 60%, 65%, 70%, 75%, 80%, 85%, 90%, 95%, 96%, 97%, 98%, 99%or 100% or any value or range therein, as compared to a control.

In some embodiments, a method for increasing abiotic stress tolerance of a plant or part thereof is provided, the method comprising applying a composition comprising an effective amount of a fungal mycelia extract to a plant or plant part thereof, wherein the extract comprises 6-oxopiperidine-2- carboxylate and/or piperidine, and or an analogue thereof, a salt thereof, or any combination thereof, thereby increasing the abiotic stress tolerance of the plant or part thereof as compared to a control plant or part thereof (e.g., a plant or part thereof to which the composition or extract of the invention is has not been applied). In some embodiments, the method comprises applying the composition at least once (e.g., about 1, 2, 3, 4, 5, 6, 7, 8, 9, 10, 11, 12 or more times). In some embodiments, the method comprises applying the composition at least two times (e.g., about 2, 3, 4, 5, 6, 7, 8, 9, 10, 11, 12 or more times).

In some embodiments, abiotic stress may include, but is not limited to, drought, salinity (e.g., medium salinity (ECₑ=4-8 dSm⁻¹); high salinity (ECₑ>8 dSm⁻¹), flooding, freezing (e.g., about 0°C or less), chilling or cold temperature (e.g., less than about 10-15°C), heat or high temperatures (e.g., more than about 40°C), high light intensity (e.g. more than about 10,000 foot candles), low light intensity (e.g. less than about 1000 foot candles), and/or ozone, and/or combinations thereof. In some embodiments, the abiotic stress is drought. In some embodiments, the abiotic stress is salinity.

As used herein, "an "increased tolerance to abiotic stress" or "increased resistance" to abiotic stress" are used interchangeably and refer to the ability of a plant or part thereof exposed to abiotic stress and contacted with a composition of the invention to withstand a given abiotic stress better than a control plant or part thereof (i.e., a plant or part thereof that has been exposed to the same abiotic stress but has not been contacted with a composition comprising a fungal mycelia extract comprising piperidine and/or an analogue and/or salt thereof). Increased tolerance to abiotic stress can be measured using a variety of parameters including, but not limited to, the size and number of plants or parts thereof, and the like (e.g., number and size of fruits), the level or amount of cell division, the amount of floral abortion, the amount of sunburn damage, crop yield, and the like. Thus, in some embodiments of this invention, a plant or part thereof having been contacted with a composition of the present invention, and having increased tolerance to the abiotic stress, for example, would have increased fruit/seed number and/or weight as compared to a plant or part thereof exposed to the same stress but not having been contacted with said composition.

In some embodiments, the percent increase in resistance/tolerance to abiotic stress as compared to a control may be an increase in a range from about 0.1% to about 100%. In some embodiments, the percent increase in resistance/tolerance to disease may be in a range from about 0.1% to about 10%, 0.1% to about 30%, about 0.1% to about 50%, about 0.1% to about 80%, about 0.1% to about 90%, about 0.1% to about 95%, about 1% to about 10%, about 1% to about 20%, about 1% to about 40%, about 1% to about 50%, about 1% to about 75%, about 1% to about 95%, about 1% to about 100%, about 10% to about 20%, about 10% to about 40%, about 10% to about 50%, about 10% to about 70%, about 10% to about 80%, about 10% to about 90%, about 10% to about 100%, about 20% to about 40%, about 20% to about 75%, about 20% to about 90%, about 20% to about 95%, about 20% to about 100%, about 25% to about 50%, about 50% to about 75%, about 50% to about 95%, about 50% to about 100% , about 75% to about 90%, about 75% to about 100%, about 90% to about 95%, about 90% to about 100% or any value or range therein, as compared to a control. In some embodiments, the percent increase in resistance/tolerance to abiotic stress may be about 0.01%, 0.05%, 0.1%, 0.5%, 1%, 1.5%, 2%, 2.5%, 3, 3.5%, 4%, 4.5%, 5%, 5.5%, 6%, 6.5%, 7%, 7.5%, 8%, 8.5%, 9%, 9.5%, 10%, 11%, 12%, 13%, 14%, 15%, 16%, 17, 5, 18%, 19%, 20%, 21%, 22%, 23%, 24%, 25%, 26%, 27%, 28%, 29%, 30%, 35%, 40%, 45%, or 50%, 55%, 60%, 65%, 70%, 75%, 80%, 85%, 90%, 95%, 96%, 97%, 98%, 99% or 100% or any value or range therein, as compared to a control.

An extract or a composition of the invention may be applied to any plant or part thereof. Thus, an extract or a composition of the invention may be applied to a variety of plants in various forms or sites, such as, e.g., foliage, buds, flowers, fruits, ears or spikes, seeds, bulbs, stem tubers, roots and seedlings. As used herein, bulbs mean discoid stem, rhizomes, root tubers, and rhizophores. In the present disclosure, an extract or composition of the invention may also be applied to cuttings (e.g., sugar cane stem cuttings).

As used herein, "a plant" means any monocotyledonous and dicotyledonous plant, and any annual and perennial dicotyledonous and monocotyledonous plant. Example plants include, but are not limited to, those of the genera *Glycine, Vitis, Asparagus, Populus, Pennisetum, Lolium, Oryza, Zea, Avena, Hordeum, Secale, Triticum, Helianthus, Gossypium, Medicago, Pisum, Acer, Actinidia, Abelmoschus, Agropyron, Allium, Amaranthus, Apium, Arachis, Asparagus, Beta, Brassica, Camellia, Canna, Capsicum, Carex, Carica papaya, Carya, Castanea, Cinnamomum, Citrullus, Citrus, Cocos, Coffea, Colocasia, Cola, Coriandrum, Corylus, Crataegus, Crocus, Cucurbita, Cucumis, Cynara, Daucus, Desmodium, Dimocarpus, Dioscorea, Diospyros, Echinochloa, Elaeis, Eleusine, Eriobotrya, Eugenia, Fagopyrum, Fagus, Ficus, Fortunella, Fragaria, Ginkgo, Hemerocallis, Hibiscus, Ipomoea, Juglans, Lactuca, Lathyrus, Lens, Linum, Litchi, Lotus, Lupinus, Luzula, Malus, Malpighia, Mammea, Mangifera, Manihot, Manilkara, Medicago, Melilotus, Mentha, Miscanthus, Musa, Nicotiana, Olea, Opuntia, Ornithopus, Panicum, Passiflora, Persea, Phaseolus, Pinus, Pistacia, Pisum, Poa, Prosopis, Prunus, Quercus, Raphanus, Rheum, Ribe, Rubus, Sambucus, Secale, Sesamum, Sinapis, Solanum, Sorghum, Spinacia, Tamarindus, Theobroma, Trifolium, Tropaeolum, Vaccinium, Vigna, Vitis, Zizania* or *Ziziphus Sorghum, Saccharum* and *Lycopersicum,* or the class Liliatae. In some embodiments, a plant or part thereof is from the genera *Glycine, Vitis, Asparagus, Populus, Pennisetum, Lolium, Oryza, Zea, Avena, Hordeum, Secale, Triticum, Sorghum, Saccharum* and *Lycopersicum,* or the class Liliaceae.

As used herein, a "plant" includes mature plants, seeds, shoots and seedlings, plant parts, propagation material, plant organs, tissue, protoplasts, callus and other cultures, for example cell cultures derived from the above, and all other types of associations of plant cells which give functional or structural units. A "mature plant" refers to a plant at any developmental stage beyond the seedling stage and includes, but is not limited, to an adult or mature plant, a budding plant, a flowering plant, and/or a fruiting plant. "Seedling" refers to a young, immature plant in an early developmental stage. The term "plant part," as used herein, includes, but is not limited to, reproductive tissues (*e.g*., petals, sepals, stamens, pistils, receptacles, anthers, pollen, flowers, fruits, flower buds, ovules, seeds, embryos, nuts, kernels, ears, cobs and husks); vegetative tissues (*e.g*., petioles, stems, roots, root hairs, root tips, pith, coleoptiles, stalks, shoots, branches, bark, apical meristem, axillary bud, cotyledon, hypocotyls, and leaves); vascular tissues (*e.g.*, phloem and xylem); specialized cells such as epidermal cells, parenchyma cells, collenchyma cells, sclerenchyma cells, stomata, guard cells, cuticle, mesophyll cells; callus tissue; and cuttings. The term "plant part" also includes plant cells, including plant cells that are intact in plants and/or parts of plants, plant protoplasts, plant tissues, plant organs, plant cell tissue cultures, plant calli, plant clumps, and the like. As used herein, "shoot" refers to the above ground parts including the leaves and stems. As used herein, the term "tissue culture" encompasses cultures of tissue, cells, protoplasts and callus.

As used herein, "plant propagation material" or "plant propagating material" refers to any plant material from which a plant or plant part can be derived. In some embodiments, plant propagation material includes, but is not limited to, seeds, seedlings, young plants, cuttings, cell suspensions, protoplasts, callus culture, tissue culture, protocorms, explants, germplasm, bulbs and/or tubers, or any combination thereof.

A variety of seeds or bulbs may be used in the methods described herein including but are not limited to plants in the families *Solanaceae* and *Cucurbitaceae,* as well as plants selected from *Calibrachoa, Capsicum, Nicotiana, Nierembergia, Petunia, Solanum, Brassica, Cucurbita, Cucumis, Citrullus, Glycine,* such as *Glycine max* (Soy), *Calibrachoa* × *hybrida, Capsicum annuum* (pepper), *Nicotiana tabacum* (tobacco), *Nierenbergia scoparia* (cupflower), *Petunia, Solanumlycopersicum* (tomato), *Solanum tuberosum* (potato), *Solanum melongena* (eggplant), *Cucurbita maxima* (squash), *Cucurbita pepo* (pumpkin, zucchini), *Cucumis metuliferus* (Horned melon) *Cucumis melo* (Musk melon), *Cucumis sativus* (cucumber) and *Citrullus lanatus* (watermelon). Various monocotyledonous plants, in particular those which belong to the family Poaceae, may be used with the methods described herein, including but not limited to, *Hordeum, Avena, Secale, Triticum, Sorghum, Zea, Saccharum, Oryza, Hordeum vulgare* (barley), *Triticum aestivum* (wheat), *Triticum aestivum* subsp. *spelta* (spelt), *Triticale, Avena sativa* (oats), *Secale cereale* (rye), *Sorghum bicolor* (sorghum), *Zea mays* (maize), *Saccharum officinarum* (sugarcane) and *Oryza sativa* (rice).

Additional examples of plants for which health and/or performance may be improved using the methods described herein include the following crops: rice, corn, canola, soybean, wheat, buckwheat, beet, rapeseed, sunflower, sugar cane, tobacco, and pea, etc.; vegetables: solanaceous vegetables such as paprika and potato; cucurbitaceous vegetables; cruciferous vegetables such as Japanese radish, white turnip, horseradish, kohlrabi, Chinese cabbage, cabbage, leaf mustard, broccoli, and cauliflower, asteraceous vegetables such as burdock, crown daisy, artichoke, and lettuce; liliaceous vegetables such as green onion, onion, garlic, and asparagus; ammiaceous vegetables such as carrot, parsley, celery, and parsnip; chenopodiaceous vegetables such as spinach, Swiss chard; lamiaceous vegetables such as *Perilla frutescens,* mint, basil; strawberry, sweet potato, *Dioscorea japonica,* colocasia; flowers; foliage plants; grasses; fruits: pomaceous fruits (apple, pear, Japanese pear, Chinese quince, quince, etc.), stone fleshy fruits (peach, plum, nectarine, *Prunus mume,* cherry fruit, apricot, prune, etc.), citrus fruits (*Citrus unshiu,* orange, tangerine, lemon, lime, grapefruit, etc.), nuts (chestnuts, walnuts, hazelnuts, almond, pistachio, cashew nuts, macadamia nuts, etc.), berries (blueberry, cranberry, blackberry, raspberry, etc.), grape, kaki fruit, olive, Japanese plum, banana, coffee, date palm, coconuts, etc.; and trees other than fruit trees; tea, mulberry, flowering plant, roadside trees (ash, birch, dogwood, Eucalyptus, *Ginkgo biloba,* lilac, maple, *Quercus,* poplar, Judas tree, *Liquidambar formosana,* plane tree, zelkova, Japanese arborvitae, fir wood, hemlock, juniper, *Pinus, Picea,* and *Taxus cuspidata*).

An embodiment of the present disclosure further provides for plants having increased fruit production compared to plants where the composition of the present disclosure has not been applied.

An embodiment of the present disclosure further provides for plants having increased inflorescence production compared to plants where the composition of the present disclosure has not been applied.

An embodiment of the present disclosure further provides for plants having increased fruit quality when compared to plants where the composition of the present disclosure has not been applied.

An embodiment of the present disclosure further provides for plants having increased tolerance to fungi when compared to plants where the composition of the present disclosure has not been applied.

An embodiment of the present disclosure further provides for plants having increased tolerance to white mold *(Sclerotinia sclerotiorum)* when compared to plants where the composition of the present disclosure has not been applied.

An embodiment of the present disclosure further provides for plants having increased tolerance to *Botrytis cinerea* when compared to plants where the composition of the present disclosure has not been applied.

An embodiment of the present disclosure further provides for plants having increased tolerance to disease when treated with the composition of the present disclosure in combination with beneficial microbes when compared to untreated plants.

An embodiment of the present disclosure further provides for plants having increased tolerance to fungi when treated with the composition of the present disclosure in combination with beneficial microbes when compared to untreated plants.

An embodiment of the present disclosure further provides for plants having increased tolerance to white mold (*Sclerotinia sclerotiorum)* when treated with the composition of the present disclosure in combination with *Bacillus subtilis* when compared to untreated plants.

An embodiment of the present disclosure further provides for plants having increased tolerance to plant viruses when compared to plants where the composition of the present disclosure has not been applied.

An embodiment of the present disclosure further provides for plants having decreased viral load when compared to plants where the composition of the present disclosure has not been applied.

An embodiment of the present disclosure further provides for plants having increased tolerance to tomato leaf curl New Dehli virus (ToLCNDV) compared to plants where the composition of the present disclosure has not been applied.

A fungal mycelia extract (PRBT) may be applied as a soil treatment in the form of a solid or a liquid. Thus in some embodiments, the composition may be applied as a spray onto soil, soil incorporation, and/or perfusion of a chemical liquid into the soil (irrigation of chemical liquid, soil injection, and dripping of chemical liquid). The placement of PRBT during soil treatment includes, but is not limited to, planting hole, furrow, around a planting hole, around a furrow, entire surface of cultivation lands, the parts between the soil and the plant, area between roots, area beneath the trunk, main furrow, growing box, seedling raising tray and seedbed, seedling raising. PRBT soil treatment may be before seeding, at the time of seeding, immediately after seeding, raising period, before settled planting, at the time of settled planting, and/or growing period after settled planting.

Alternatively, an irrigation liquid may be mixed with PRBT in advance and, for example, used for treatment by an appropriate irrigating method including the irrigation method mentioned above and the other methods such as sprinkling and flooding. PRBT may also be applied by winding a crop with a resin formulation processed into a sheet or a string, putting a string of the resin formulation around a crop so that the crop is surrounded by the string, and/or laying a sheet of the resin formulation on the soil surface near the root of a crop.

In another embodiment, PRBT may be used for treating seeds or bulbs as well as a PRBT spraying treatment for seeds in which a suspension of PRBT is atomized and sprayed on a seed surface or bulb surface. A smearing treatment may also be used, for example, where a wettable powder, an emulsion or a flowable agent of the PRBT is applied to seeds or bulbs with a small amount of water added or applied as is without dilution. In addition, an immersing treatment may be used in which seeds are immersed in a solution of PRBT for a certain period of time, film coating treatment, and pellet coating treatment.

PRBT may be used for the treatment of seedlings, including spraying treatment comprised of spraying the entire seedlings with a dilution having a proper concentration of active ingredients prepared by diluting PRBT with water. As with seed treatment, an immersing treatment may also be used comprised of immersing seedlings in the dilution, and coating treatment of adhering PRBT formulated into a dust formulation to the entire seedlings.

Soil may be treated/contacted with PRBT before and/or after sowing seedlings including spraying a dilution having a proper concentration of active ingredients prepared by diluting PRBT with water and applying the mixture to seedlings or the soil around seedlings after sowing seedlings. A spray treatment of PRBT formulated into a solid formulation such as a granule to soil around seedlings at sowing seedlings may also be used.

When PRBT may be applied as a treatment to foliage, floral organs or ears or spikes of plants, such as foliage spraying; treatment of seeds, such as seed sterilization, seed immersion or seed coating; treatment of seedlings; treatment of bulbs; and treatment of cultivation lands of plants, such as soil treatment. PRBT may be applied only to specific sites of plants, such as floral organ in the blooming season including before blooming, during blooming and after blooming, and the ear or spike in the earing season, or may be applied to entire plants.

The invention will now be described with reference to the following examples. It should be appreciated that these examples are not intended to limit the scope of the claims to the invention, but are rather intended to be exemplary of certain embodiments. Any variations in the exemplified methods that occur to the skilled artisan are intended to fall within the scope of the invention.

### EXAMPLES

The following examples are provided to illustrate further the various applications and are not intended to limit the invention beyond the limitations set forth in the appended claims. Any variations in the exemplified methods that occur to the skilled artisan are intended to fall within the scope of the invention.

### Example 1 - PRBT results in increased metabolites in Arabidopsis and Tomato

For each metabolomics analysis, wild type (Col-0) seeds of *Arabidopsis thaliana* or Money Maker seeds of tomato were sown in plastic pots. Plants were grown for 3 weeks under short day (10 hours light, 14 hours dark, 21 °C light and 20 °C night, 65% humidity) conditions. After 4 weeks plants were sprayed with a formulated fungal mycelia extract (PRBT with surfactant 2:1 v/v) at the rate of 0.054 g L⁻¹ (0.5 ml/plant). Plant material was harvested and lyophilized 8 days later from individual plants (n=20). A broad metabolite coverage analysis was performed to determine the main metabolic differences between control plants and plants treated with the mycelial extract. Samples were prepared using the automated MicroLab STAR system from Hamilton Company. Several recovery standards were added prior to the first step in the extraction process for QC purposes. To remove protein, dissociate small molecules bound to protein or trapped in the precipitated protein matrix, and to recover chemically diverse metabolites, proteins were precipitated with methanol under vigorous shaking for 2 min (Glen Mills GenoGrinder 2000) followed by centrifugation. The resulting extract was divided into four fractions: two were analyzed by two separate reverse phase (RP)/UPLC-MS/MS methods with positive ion mode electrospray ionization (ESI), one by RP/UPLC-MS/MS with negative ion mode ESI, and one by HILIC/UPLC-MS/MS with negative ion mode ESI. A total of 373 biochemicals of known identity were determined. Following log transformation and imputation of missing values, if any, with the minimum observed value for each compound, ANOVA contrasts were used to identify biochemicals that differed significantly between the control and the PRBT treated plants.

Shown below in Table 1 are biochemicals (column 1) that differed significantly (P <0.05) between the control and the PRBT treated plants that were common among Arabidopsis and tomato. The PubChem compound identification number is shown in column 2 for each biochemical. The fold change of the PRBT treated vs the control treated plants (column 4), the ANOVA p-value (column 5) as well as the mean values for each biochemical (columns 6 and 7) are indicated.

**Table 1A -PRBT results in increased metabolites in Arabidopsis and Tomato**

| | | | **Fold of Change** | **Statistical Values** | **Mean Values** | |
|---|---|---|---|---|---|---|
| | | | | **PRBT / CONTROL*** | | |
| **Biochemical Name** | **PUBCHEM** | **PLANT** | **PRBT / CONTROL** | **p-value** | **CONTROL** | **PRBT** |
| 6-oxopiperidine-2-carboxylate | 3014237 | Tomato | **33.09** | 2.90E-07 | 0.0763 | 2.5248 |
| | | Arabidopsis | **9.17** | 0.0004 | 0.7142 | 6.5469 |
| stachydrine | 115244 | Tomato | **5.83** | 1.66E-05 | 0.1763 | 1.0282 |
| | | Arabidopsis | **1.39** | 0.0222 | 1.0721 | 1.4909 |
| mannitol/sorbitol | 5780 | Tomato | **2.33** | 0.0002 | 0.6718 | 1.5661 |
| | | Arabidopsis | **2.33** | 0.0017 | 0.7872 | 1.8332 |

As shown above in Table 1, application of PRBT increases the levels of 6-oxopiperidine-2-carboxylate, the stachydrine and the mannitol/sorbitol ratio.

### Example 2 - PRBT induces transcriptomic changes in Arabidopsis

For each transcriptomic analysis wild type (Col-0) seeds of *Arabidopsis thaliana* were sown in plastic pots. Plants were grown for 3 weeks under short day (10 hours light, 14 hours dark, 21 °C light and 20 °C night, 65% humidity) conditions. After 3 weeks plants were sprayed with a formulated fungal mycelia extract (PRBT) at the rate of 0.054 g L⁻¹(0.5 ml/plant). Plant material was harvested and lyophilized 20 days later from individual plants (n=20). RNA sequencing experiment was performed on 4 samples. Prior to further analysis, a quality check was performed on the raw sequencing data by using FastQC, then low quality portions of the reads were removed with BBDuk. The minimum length of the reads after trimming was set to 35 bp and the minimum base quality score to 25. Once the quality of the replicates was checked, an analysis was performed to identify the genes that are differentially expressed between several groups of samples. Genes were considered significantly differentially expressed if the p-value corrected for multiple testing (Benjamini-Hochberg) (FDR) of the statistical test was less or equal to 0.05. In addition, a filter to the fold changes was applied in order to retain only the genes with a logarithmic fold change higher than 0.5 or less than -0.5. There were 2596 genes that were significantly (P <0.05) differently expressed between the PRBT treated and the control plants, out of which 1465 were up regulated, while 1131 were down regulated.

Among the up regulated genes there are genes related with photosynthesis (ATCG00280;ATCG00680, ATCG00550, ATCG00070, ATCG00080, ATCG00580, ATCG00270, AT1G29930, AT2G34430, ATCG00020, AT1G29910, ATCG00350, ATCG00340, ATCG00280, ATCG00680, AT1G29920, ATCG00730, ATCG00280, AT2G01918, ATCG00680, ATCG00580), response to salt stress (AT5G58580), nutrients transport and binding (AT4G33000, AT1G21840, AT2G47400, AT1G76560, AT4G09640, AT4G13800, AT3G23870, AT3G26740, AT5G57345, AT5G27290), positive regulation of cellular response to phosphate starvation (AT5G20150), hormones transport (AT1G17140, AT5G64770, AT4G09460, AT4G35770, AT5G67480, AT4G12030, AT1G18710, AT5G37260, AT5G44420, AT1G74430, AT5G60890, AT1G08810, AT1G57560, AT5G39610, AT4G27410, AT1G66760, AT3G16770, AT3G09600, AT2G16720, AT1G49010, AT3G48330, AT4G26400, AT2G42530, AT2G23430, AT4G39260, AT5G37260, AT5G53160, AT4G00310, AT3G17510, AT1G22640, AT5G59220, AT1G08810, AT4G15910, AT5G15970, AT3G22060, AT5G39610, AT4G27410, AT4G34000, AT1G16060, AT1G68670, AT3G09600, AT2G23840, AT2G32690, AT2G16720, AT1G73480, AT2G26980, AT2G40190), and fruit morphogenesis (AT5G45710).

**Table 1** below shows a list of genes that are upregulated in PRBT treated Arabidopsis plants compared to untreated plants. Column 1 shows the gene ontology identification (GO ID), columns 2 and 3 list the type and description of the genes respectively, column 4 shows the P-value, column 5 shows the enrichment score (enrichment fold of the corresponding GO term in the list of differentially expressed genes respect to the reference genome), and column 7 shows the genetic loci.

**Table 1B - PRBT treatment upregulates genes in Arabidopsis**

| ID | Type | Description | P-value | Enrichment Score | Loci |
|---|---|---|---|---|---|
| GO:0097036 | biological process | regulation of plasma membrane sterol distribution | 0.00E+00 | 20.78 | AT4G01510; AT1G01020 |
| GO:0035061 | cellular component | interchromatin granule | 0.00E+00 | 20.78 | AT1G09140; AT1G02840 |
| GO:0018345 | biological process | protein palmitoylation | 0.00E+00 | 20.78 | AT3G63420; AT3G22942 |
| GO:0009521 | cellular component | photosystem | 0.00E+00 | 20.78 | ATCG00280; ATCG00680 |
| GO:0032366 | biological process | intracellular sterol transport | 0.00E+00 | 20.78 | AT4G01510; AT1G01020 |
| GO:0047158 | molecular function | sinapoylglucose-sinapoylglucose O-sinapoyltransferase activity | 1.11E-04 | 13.85 | AT2G22980; AT2G22990 |
| GO:0009443 | biological process | pvridoxal 5-phosphate salvage | 1.11E-04 | 13.85 | AT5G53580; AT5G37850 |
| GO:0032541 | cellular component | cortical endoplasmic reticulum | 1.11E-04 | 13.85 | AT4G01510; AT1G01020 |
| GO:0080153 | biological process | negative regulation of reductive pentosephosphate cycle | 1.11E-04 | 13.85 | AT2G47400; AT1G76560 |
| GO:0045723 | biological process | positive regulation of fatty acid biosynthetic process | 1.11E-04 | 13.85 | AT1G79700; AT1G16060 |
| GO:1901959 | biological process | positive regulation of cutin biosynthetic process | 1.11E-04 | 13.85 | AT1G79700; AT1G16060 |
| GO:0009772 | biological process | photosynthetic electron transport in photosystem II | 9.10E-07 | 11.54 | ATCG00270; ATCG00020; AT1G60600; ATCG00280; A TCG00680 |
| GO:0006501 | biological process | C-terminal protein lipidation | 7.41E-05 | 10.39 | AT5G17290; AT3G13970; AT1G54210 |
| GO:0006376 | biological process | mRNA splice site selection | 4.29E-04 | 10.39 | AT1G09140; AT5G17440 |
| GO:0033617 | biological process | mitochondrial respiratory chain complex IV assembly | 4.29E-04 | 10.39 | AT1G66590; AT4G14615 |
| GO:0006369 | biological process | termination of RNA polymerase II transcription | 4.29E-04 | 10.39 | AT1G66500; AT5G43620 |
| GO:1901000 | biological process | regulation of response to salt stress | 2.31E-03 | 10.39 | AT5G58580 |
| GO:0005534 | molecular function | galactose binding | 2.31E-03 | 10.39 | AT2G42530 |
| GO:0033506 | biological process | glucosinolate biosynthetic process from homomethionine | 2.31E-03 | 10.39 | AT4G12030 |
| GO:0071454 | biological process | cellular response to anoxia | 2.31E-03 | 10.39 | AT1G76560 |
| GO:0010028 | biological process | xanthophyll cycle | 2.31E-03 | 10.39 | AT1G08550 |
| GO:0051973 | biological process | positive regulation of telomerase activity | 2.31E-03 | 10.39 | AT3G09290 |
| GO:0043266 | biological process | regulation of potassium ion transport | 2.31E-03 | 10.39 | AT4G33000 |
| GO:0045694 | biological process | regulation of embryo sac egg cell differentiation | 2.31E-03 | 10.39 | AT5G06160 |
| GO:0008202 | biological process | steroid metabolic process | 2.31E-03 | 10.39 | AT2G38050 |
| GO:0006637 | biological process | acyl-CoA metabolic process | 2.31E-03 | 10.39 | AT4G00520 |
| GO:1900384 | biological process | regulation of flavonol biosvnthetic process | 2.31E-03 | 10.39 | AT2G16720 |
| GO:0009539 | cellular component | photosystem II reaction center | 2.74E-05 | 9.23 | ATCG00550; ATCG00070; ATCG00080; ATCG00580 |
| GO:0016151 | molecular function | nickel cation binding | 1.66E-04 | 8.90 | AT1G21840; AT2G47400; AT1G76560 |
| GO:2000012 | biological process | regulation of auxin polar transport | 1.03E-03 | 8.31 | AT1G17140; AT5G64770 |
| GO:0022622 | biological process | root system development | 1.03E-03 | 8.31 | AT5G64770; AT5G51451 |
| GO:0009512 | cellular component | cytochrome b6f complex | 1.03E-03 | 8.31 | ATCG00210; AT2G26500 |
| GO:0042372 | biological process | phylloquinone biosynthetic process | 5.54E-04 | 6.93 | AT1G23360; AT1G60550; AT1G60600 |
| GO:0019104 | molecular function | DNA N-glycosylase activity | 5.54E-04 | 6.93 | AT1G05900; AT4G34060; AT1G52500 |
| GO:0032447 | biological process | protein urmylation | 1.99E-03 | 6.93 | AT2G45695; AT1G76170 |
| GO:2000038 | biological process | regulation of stomatal complex development | 6.72E-03 | 6.93 | AT4G12970 |
| GO:0080183 | biological process | response to photooxidative stress | 6.72E-03 | 6.93 | AT1G03850 |
| GO:0015086 | molecular function | cadmium ion transmembrane transporter activity | 6.72E-03 | 6.93 | AT1G15960 |
| GO:2000123 | biological process | positive regulation of stomatal complex development | 6.72E-03 | 6.93 | AT4G12970 |
| GO:0051457 | biological process | maintenance of protein location in nucleus | 6.72E-03 | 6.93 | AT5G02200 |
| GO:0010321 | biological process | regulation of vegetative phase change | 6.72E-03 | 6.93 | AT2G33810 |
| GO:0071452 | biological process | cellular response to singlet oxygen | 6.72E-03 | 6.93 | AT3G02790 |
| GO:0071457 | biological process | cellular response to ozone | 6.72E-03 | 6.93 | AT5G18100 |
| GO:0010483 | biological process | pollen tube reception | 6.72E-03 | 6.93 | AT2G17430 |
| GO:0080040 | biological process | positive regulation of cellular response to phosphate starvation | 6.72E-03 | 6.93 | AT5G20150 |
| GO:0070574 | biological process | cadmium ion transmembrane transport | 6.72E-03 | 6.93 | AT1G15960 |
| GO:1901601 | biological process | strigolactone biosvnthetic process | 6.72E-03 | 6.93 | AT1G03055 |
| GO:0004475 | molecular function | mannose-1-phosphate guanylyltransferase activity | 6.72E-03 | 6.93 | AT4G30570 |
| GO:0048530 | biological process | fruit morphogenesis | 6.72E-03 | 6.93 | AT5G45710 |
| GO:0006672 | biological process | ceramide metabolic process | 6.72E-03 | 6.93 | AT1G71190 |
| GO:0016531 | molecular function | copper chaperone activity | 6.72E-03 | 6.93 | AT1G53030 |
| GO:1900033 | biological process | negative regulation of trichome patterning | 6.72E-03 | 6.93 | AT2G30424 |
| GO:0016168 | molecular function | chlorophyll binding | 3.16E-07 | 6.11 | ATCG00270; AT1G29930; AT2G34430; ATCG00020; AT1G29910; ATCG00350; ATCG00340; ATCG00280. ATCG00680; AT1G29920 |
| GO:0009767 | biological process | photosynthetic electron transport chain | 9.61E-05 | 6.11 | ATCG00730; ATCG00280; AT2G01918; ATCG00680; ATCG00580 |
| GO:0042594 | biological process | response to starvation | 3.36E-03 | 5.94 | AT5G17290; AT5G54730 |
| GO:0016125 | biological process | sterol metabolic process | 3.36E-03 | 5.94 | AT4G01510; AT1G01020 |
| GO:0080110 | biological process | sporopollenin biosvnthetic process | 3.36E-03 | 5.94 | AT1G02050; AT4G35420 |
| GO:0009523 | cellular component | photosystem II | 9.91E-12 | 5.85 | ATCG00270; AT1G29930; AT2G34430; ATCG00020; AT1G29910; AT2G39470; AT3G55330; AT2G30790; AT2G28605; ATCG00550; ATCG00280; AT2G01918; AT1G77090; ATCG00710; ATCG00070; ATCG00680; AT1G29920; AT4G19100; ATCG00080; ATCG00580 |
| GO:0015693 | biological process | magnesium ion transport | 1.34E-03 | 5.67 | AT4G09640; AT4G13800; AT3G23870 |
| GO:0009654 | cellular component | photosystem II oxygen evolving complex | 7.31E-05 | 5.42 | AT2G39470; AT3G55330; AT2G30790; AT2G28605; AT2G01918; AT1G77090 |
| GO:0000303 | biological process | response to superoxide | 1.94E-03 | 5.19 | AT4G26400; AT5G39610; AT4G13820 |
| GO:0005834 | cellular component | heterotrimeric G-protein complex | 5.19E-03 | 5.19 | AT3G63420; AT3G22942 |
| GO:0008200 | molecular function | ion channel inhibitor activity | 5.19E-03 | 5.19 | AT2G43510; AT2G43530 |
| GO:0071702 | biological process | organic substance transport | 1.30E-02 | 5.19 | AT4G12030 |
| GO:0071470 | biological process | cellular response to osmotic stress | 1.30E-02 | 5.19 | AT5G58580 |
| GO:0009759 | biological process | indole glucosinolate biosvnthetic process | 1.30E-02 | 5.19 | AT5G60890 |
| GO:0047274 | molecular function | galactinol-sucrose galactosyltransferase activity | 1.30E-02 | 5.19 | AT1G55740 |
| GO:0048629 | biological process | trichome patterning | 1.30E-02 | 5.19 | AT2G30424 |
| GO:0006490 | biological process | oligosaccharide-lipid intermediate biosynthetic process | 1.30E-02 | 5.19 | AT2G40190 |
| GO:0032922 | biological process | circadian regulation of gene expression | 1.30E-02 | 5.19 | AT3G09600 |
| GO:0071484 | biological process | cellular response to light intensity | 1.30E-02 | 5.19 | AT5G18100 |
| GO:1903825 | biological process | organic acid transmembrane transport | 1.30E-02 | 5.19 | AT4G12030 |
| GO:0009531 | cellular component | secondary cell wall | 1.30E-02 | 5.19 | AT5G62880 |
| GO:0035197 | molecular function | siRNA binding | 1.30E-02 | 5.19 | AT2G32940 |
| GO:0009969 | biological process | xyloglucan biosynthetic process | 1.30E-02 | 5.19 | AT1G64440 |
| GO:0043617 | biological process | cellular response to sucrose starvation | 1.30E-02 | 5.19 | AT3G13450 |
| GO:0071398 | biological process | cellular response to fatty acid | 1.30E-02 | 5.19 | AT1G12010 |
| GO:0009522 | cellular component | photosystem I | 1.45E-05 | 4.67 | ATCG01060; AT1G29930; AT2G34430; AT1G29910; ATCG00520; ATCG00350; ATCG00340; ATCG00630; AT1G29920 |
| GO:0009743 | biological process | response to carbohydrate | 7.51E-03 | 4.62 | AT4G26400; AT5G39610 |
| GO:0043068 | biological process | positive regulation of programmed cell death | 1.03E-02 | 4.16 | AT5G06100; AT1G32540 |
| GO:0010497 | biological process | plasmodesmata-mediated intercellular transport | 1.03E-02 | 4.16 | AT1G04520; AT2G33330 |
| GO:0009765 | biological process | photosynthesis, light harvesting | 4.17E-03 | 3.61 | AT1G29930; AT2G34430; AT1G29910; AT1G29920 |
| GO:0015979 | biological process | photosynthesis | 4.76E-10 | 3.59 | ATCG01060; ATCG00270; AT1G29930; AT2G34430; AT2G04039; AT1G29910; ATCG00520; ATCG00540; ATCG00350; AT2G39470; AT3G55330; ATCG00340; ATCG00630; ATCG00490; AT2G30790; AT2G28605; ATCG00550; ATCG00280; AT2G01918; AT1G77090; ATCG00210; ATCG00710; ATCG00070; ATCG00680; AT1G29920; AT4G19100; ATCG00720; ATCG00080; ATCG00580 |
| GO:0006661 | biological process | phosphatidylinositol biosynthetic process | 2.56E-05 | 3.56 | AT4G01510; AT1G21840; AT1G78790; AT1G79070; AT1G56260; AT5G56520; AT2G21180; AT1G28070; AT3G21260; AT5G24170; AT1G78480; AT4G31560 |
| GO:0015095 | molecular function | magnesium ion transmembrane transporter activity | 1.16E-02 | 3.28 | AT4G09640; AT4G13800; AT3G23870 |
| GO:0042651 | cellular component | thylakoid membrane | 1.16E-02 | 3.28 | ATCG01060; ATCG00730; AT4G31560 |
| GO:0009958 | biological process | positive gravitropism | 7.24E-03 | 3.20 | AT5G64770; AT5G62500; AT5G45710; AT3G20130 |
| GO:0009750 | biological process | response to fructose | 2.19E-03 | 2.97 | AT2G39570; AT4G10910; AT3G61060; AT2G42900; AT1G74670; AT2G17880; AT4G23870 |
| GO:0019684 | biological process | photosynthesis, light reaction | 2.49E-03 | 2.91 | ATCG00270; ATCG00020; ATCG00280; ATCG00680; ATCG01100; ATCG00580; ATCG00420 |
| GO:0008422 | molecular function | beta-glucosidase activity | 4.73E-03 | 2.83 | AT3G18070; AT3G62740; AT3G62750; AT1G60270; AT2G44480; AT1G60260 |
| GO:0009543 | cellular component | chloroplast thylakoid lumen | 4.73E-03 | 2.83 | AT4G26555; AT4G19830; AT3G55330; AT5G45680; AT2G28605; AT1G77090 |
| GO:0048527 | biological process | lateral root development | 8.62E-03 | 2.54 | AT2G23430; AT5G64770; AT3G63420; AT5G51451; AT5G45710; AT3G22942 |
| GO:0009535 | cellular component | chloroplast thylakoid membrane | 7.46E-09 | 2.48 | ATCG01060; ATCG00270; AT1G33810; AT2G30080; AT1G29930; AT1G51110; AT3G25480; AT2G34430; AT1G08550; AT3G17700; ATCG00020; AT1G29910; ATCG00520; ATCG00540; ATCG00350; AT2G39470; ATCG00340; ATCG00730; ATCG00140; ATCG00470; ATCG00630; ATCG00480; AT4G27700; AT2G29180; ATCG00120; ATCG00550; ATCG00280; AT2G01918; ATCG00210; ATCG00710; AT1G72640; ATCG00070; ATCG00150; AT2G01870; ATCG00680; AT1G29920; AT2G26500; ATCG0 1100; AT3G03920; AT4G19100; ATCG00720; AT3G56910; ATCG00130; ATCG00080; ATCG00420 |
| GO:0048573 | biological process | photoperiodism, flowering | 4.27E-03 | 2.28 | AT5G42820; AT1G56200; AT3G62190; AT2G42280; AT3G22420; AT1G02100; AT3G11100; AT5G04910; AT2G41250; AT3G09600 |
| GO:0009744 | biological process | response to sucrose | 8.25E-03 | 2.17 | AT2G39570; AT4G10910; AT3G61060; AT2G42900; AT1G74670; AT2G47400; AT2G17880; AT3G13450; AT4G23870 |
| GO:0009753 | biological process | response to jasmonic acid | 2.12E-03 | 1.97 | AT4G09460; AT4G35770; AT5G67480; AT4G12030; AT1G18710; AT5G37260; AT5G44420; AT1G74430; AT5G60890; AT1G08810; AT1G57560; AT5G39610; AT4G27410; AT1G66760; AT3G16770; AT3G09600; AT2G16720; AT1G49010 |
| GO:0009737 | biological process | response to abscisic acid | 6.43E-03 | 1.60 | AT3G48330; AT4G26400; AT2G42530; AT2G23430; AT4G39260; AT5G37260; AT5G53160; AT4G00310; AT3G17510; AT1G22640; AT5G59220; AT1G08810; AT4G15910; AT5G15970; AT3G22060; AT5G39610; AT4G27410; AT4G34000; AT1G16060; AT1G68670; AT3G09600; AT2G23840; AT2G32690; AT2G16720; AT1G73480; AT2G26980; AT2G40190 |
| GO:0006995 | biological process | cellular response to nitrogen starvation | 1.34E-02 | 2.77 | AT4G04620; AT5G17290; AT3G13970; AT1G54210 |
| GO:0009410 | biological process | response to xenobiotic stimulus | 1.34E-02 | 2.77 | AT3G51960; AT1G01160; AT2G42380; AT1G79880 |
| GO:0070838 | biological process | divalent metal ion transport | 1.39E-02 | 3.12 | AT3G26740; AT5G57345; AT5G27290 |
| GO:0071705 | biological process | nitrogen compound transport | 2.10E-02 | 4.16 | AT1G26440 |
| GO:0030307 | biological process | positive regulation of cell growth | 2.10E-02 | 4.16 | AT2G19690 |
| GO:0010438 | biological process | cellular response to sulfur starvation | 2.10E-02 | 4.16 | AT5G60890 |
| GO:0071333 | biological process | cellular response to glucose stimulus | 2.10E-02 | 4.16 | AT5G24800 |
| GO:0046836 | biological process | glycolipid transport | 2.10E-02 | 4.16 | AT3G21260 |
| GO:0034059 | biological process | response to anoxia | 2.10E-02 | 4.16 | AT3G11490 |
| GO:0009861 | biological process | jasmonic acid and ethylene-dependent systemic resistance | 2.10E-02 | 4.16 | AT5G44420 |
| GO:0009558 | biological process | embryo sac cellularization | 2.10E-02 | 4.16 | AT2G18390 |
| GO:0048445 | biological process | carpel morphogenesis | 2.28E-02 | 2.71 | AT4G09840; AT3G29140; AT 1G29960 |
| GO:0042538 | biological process | hyperosmotic salinity response | 2.56E-02 | 1.97 | AT3G51960; AT1G57560; AT5G39610; AT4G27410; AT5G37850; AT4G33000; AT1G73480 |
| GO:0009694 | biological process | jasmonic acid metabolic process | 2.72E-02 | 2.97 | AT1G19640; AT1G74430 |
| GO:0048232 | biological process | male gamete generation | 3.05E-02 | 3.46 | AT3G03900 |
| GO:0015976 | biological process | carbon utilization | 3.05E-02 | 3.46 | AT1G58180 |
| GO:0060918 | biological process | auxin transport | 3.05E-02 | 3.46 | AT5G56750 |
| GO:0009751 | biological process | response to salicylic acid | 3.22E-02 | 1.64 | AT4G09460; AT5G67480; AT5G37260; AT1G74430; AT1G22640; AT1G08810; AT1G57560; AT3G09600; AT4G37610; AT2G32690; AT2G16720; AT1G49010 |
| GO:0016036 | biological process | cellular response to phosphate starvation | 3.30E-02 | 1.81 | AT5G03545; AT5G41080; AT4G23000; AT1G08310; AT5G20150; AT1G73170; AT3G23870; AT1G67600 |
| GO:0010223 | biological process | secondary shoot formation | 3.90E-02 | 2.60 | AT1G03055; AT1G73870 |
| GO:0000293 | molecular function | ferric-chelate reductase activity | 4.14E-02 | 2.97 | AT5G50160 |
| GO:0019827 | biological process | stem cell population maintenance | 4.14E-02 | 2.97 | AT1G56260 |
| GO:0006817 | biological process | phosphate ion transport | 4.36E-02 | 2.23 | AT3G01970; AT5G41080; AT5G20150 |
| GO:0006970 | biological process | response to osmotic stress | 5.17E-02 | 1.66 | AT1G78290; AT3G17510; AT1G22190; AT2G21660; AT3G51960; AT5G15970; AT3G43700; AT2G40190 |
| GO:0006825 | biological process | copper ion transport | 5.34E-02 | 2.60 | AT1G53030 |
| GO:0009845 | biological process | seed germination | 6.47E-02 | 1.63 | AT3G48330; AT5G66460; AT5G37260; AT4G00310; AT3G63420; AT3G22942; AT5G06160 |
| GO:0009415 | biological process | response to water | 8.06E-02 | 2.08 | AT2G21490 |
| GO:0010204 | biological process | defense response signaling pathway, resistance gene-independent | 9.54E-02 | 1.89 | AT3G14150 |
| GO:0031408 | biological process | oxylipin biosynthetic process | 9.64E-02 | 1.81 | AT1G19640; AT3G15850 |
| GO:0006829 | biological process | zinc II ion transport | 1.11E-01 | 1.73 | AT3G58810 |

### Example 3 - PRBT increases the yield in a wide range of crops

In order to determine the plant yield productivity under normal environmental conditions, 'Sabrina' strawberry variety, "white" garlic variety , "Iceberg" lettuce variety, kohlrabi, onion, fennel and FAO700 corn plants, were grown under standard production conditions in 2013 and 120 plants of each variety per treatment (where the treatment was a control comprising standard watering and 0.054 g L⁻¹ of PRBT spray (once for garlic in combination with 0.000006 g L⁻¹ of B, 0.000012 g L⁻¹ of chelated Cu, 0.00003 g L⁻¹ of chelated Fe and 0.000012 g L⁻¹ and 0.00003 g L⁻¹ of chelated Mn; four times for lettuce, kohlrabi, onion and fennel and once for corn in combination with 0.012 g L⁻¹ of N, 0.003 g L⁻¹ of P₂O₅, 0.003 g L⁻¹ of K₂O, 0.024 g L⁻¹ of P₂O₃, 0.000006 g L⁻¹ of B, 0.000012 g L⁻¹ of chelated Cu, 0.00003 g L⁻¹ of chelated Fe and 0.000012 g L⁻¹, 0.00003 g L⁻¹ of chelated Mn, 0.00001 g L⁻¹ of chelated Mb and 0.00001 g L⁻¹ of chelated Zn) were analyzed. The control plants received the fertilizer treatments without the PRBT.

Plants were located in four different positions for each group of 30 plants from the same treatment. Fruits, leaves or roots were harvested from individual plants and total weight was determined for each plant. In order to determine the plant fruit productivity, Sabrina strawberry plants were grown under standard production conditions (from October 2013 until April 2014) and 120 plants per treatment (Control sprayed with the adjuvant or plants with PRBT (0.054 g L⁻¹) (with surfactant 2:1.5 v/w) were sprayed a total of 3 times, once every four weeks for three months were analyzed. Plants were located in four different positions for each group of 30 plants from the same treatment. Fruits were harvested from individual plants and total weight was determined for each plant.

**Table 2 - Strawberry fruit production after PRBT spray treatments every four weeks for three months**

| | **FEBRUARY** | **MARCH** | **APRIL** | **Total** |
|---|---|---|---|---|
| **CONTROL grams/plant** | 1137 | 1967 | 3221 | **6325** |
| **PRBT grams/plant** | 1361 | 3494 | 3051 | **7906** |
| **% Control** | **120** | **177** | **95** | **125** |

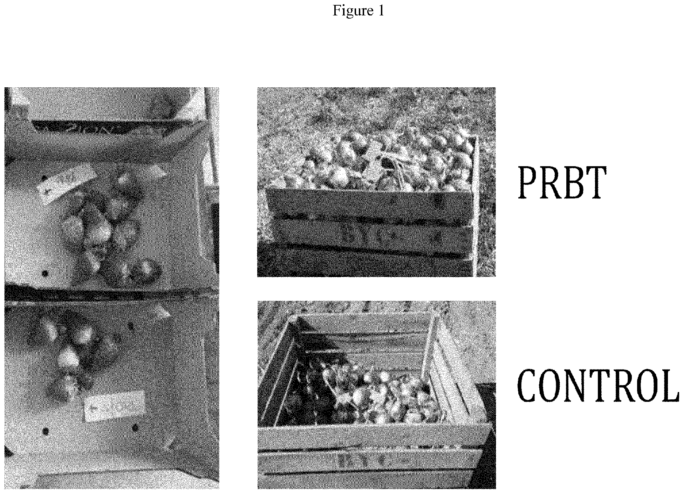

As shown in Table 2 above, strawberry fruit production increased between 20% and 77%, with an average 25% increase, when treated with PRBT compared to untreated plants. As shown in **Fig. 1** strawberry fruit production in PRBT treated plants increases by increasing yield and fruit size.

**Table 3 - Crop production increase compared to control after PRBT spray treatments**

| **Crop** | **Garlic** | **Celery** | **Kohlrabi** | **Onion** | **Fennel** | **Lettuce** | **Corn** |
|---|---|---|---|---|---|---|---|
| **% Control** | 100.1 | 103.9 | 104.8 | 110.2 | 126.9 | 105.1 | 104.1 |

As shown in Table 3 above, all crops tested showed an increase in production increased between 0.1% and 26.9%. As shown in **Fig. 1****,** onion production in PRBT treated plants increases with bigger and more onions.

### Example 4 - Broccoli plants treated with PRBT have increased inflorescence production

PRBT applied exogenously increases plant production in broccoli under normal and high salinity conditions NaCl (9mS/m). 'Parthenon' broccoli seeds were sown, grown and treated as described above. PRBT (with surfactant 2:1 v/w and antifoam) spray treatments with (0.054 g L⁻¹) at 50 ml/plant/month sprayed four times with a four week interval were analyzed. Increased plant production was measured by the average inflorescence weight and plant weight in grams. Inflorescence quality was scored where 4 corresponds to maximum quality and 1 corresponds to poor quality.

**Table 4 - Average inflorescence production and ANOVA analysis for PRBT spray treated broccoli plants under normal and high salinity growing conditions**

| IRRIGATION | N | TREATMENT | AVERAGE WEIGHT | ANOVA *P-*value | % Control |
|---|---|---|---|---|---|
| | | | (grams/ inflorescence) | | |
| NO STRESS | 120 | WATER | 362.1± 4.5 | - | 100 |
| NO STRESS | 120 | PRBT | 405.9± 3.5 | 0.0000 | 112 |
| NaCl STRESS | 120 | WATER | 234.2 ± 6.4 | - | 100 |
| NaCl STRESS | 120 | PRBT | 285.0 ± 3.8 | 0.0000 | 122 |

As shown above in **Table 4,** when treated with PRBT, broccoli plants showed an increase in inflorescence production of 12% under non stressed conditions, and an increase of 22% under high salinity conditions, compared to untreated plants.

**Table 5 - Average plant weight and ANOVA analysis for PRBT spray treated broccoli plants under normal and high salinity growing conditions**

| IRRIGATION | N | TREATMENT | AVERAGE WEIGHT | ANOVA *P-*value | % Control |
|---|---|---|---|---|---|
| | | | (grams/ plant) | | |
| NO STRESS | 120 | WATER | 977.5± 7.2 | - | 100 |
| NO STRESS | 120 | PRBT | 980.0± 8.6 | 0.0776 | 100 |
| NaCl STRESS | 120 | WATER | 869.5 ± 13 | - | 100 |
| NaCl STRESS | 120 | PRBT | 971.9 ± 92 | 0.0000 | 112 |

As shown above in **Table 5,** when treated with PRBT, broccoli plants showed an increase in plant weight of 12% under high salinity conditions compared to untreated plants.

**Table 6 - Average quality production and ANOVA analysis for PRBT spray treated broccoli plants under normal and high salinity growing conditions**

| IRRIGATION | N | TREATMENT | QUALITY SCORE | ANOVA *P-*value | % Control |
|---|---|---|---|---|---|
| | | | | | |
| NO STRESS | 120 | WATER | 3.48±0.057 | - | 100 |
| NO STRESS | 120 | PRBT | 3.71±0.045 | 0.0002 | 106 |
| NaCl STRESS | 120 | WATER | 2.84 ± 0.097 | - | 100 |
| NaCl STRESS | 120 | PRBT | 2.92 ± 0.045 | 0.3495 | 103 |

As shown above in **Table 6,** when treated with PRBT broccoli plants showed an increase in plant quality under normal and high salinity conditions compared to untreated plants.

### Example 5 - Tomato plants treated with PRBT have an increased fruit production

PRBT applied exogenously increases plant production in tomato. Tomato plants var. Mayoral were sown in a greenhouse on August 31, 2014. A total of 2244 plants were used, 1122 control plants and 1122 PRBT treated plants distributed in random blocks. Each pair of lines are separated by 1.5 meters. Within a line, the plants are separated by 50 cm. PRBT treated plants were sprayed once per month, since September 2014 to March 2015 (7 treatments) with (0.054 g L⁻¹) PRBT (with surfactant 2:1.5 v/w) at 0.36 ml/plant/month. The plants were harvested 10 times and production data were collected for each plant per harvest day.

**Table 7- Total weight tomato production for PRBT spray treated tomato plants per harvest date**

| No. of samples | Date | Total Kg production per PRBT plants | Total Kg production per CONTROL plants | % Control |
|---|---|---|---|---|
| 1122 | December 1 | 222 | 156 | 142 |
| 1122 | December 11 | 284 | 331 | 86 |
| 1122 | December 18 | 317 | 312 | 102 |
| 1122 | December 28 | 485 | 402 | 121 |
| 1122 | January 4 | 466 | 403 | 116 |
| 1122 | January 19 | 1025 | 1041 | 98 |
| 1122 | February 5 | 1897 | 1572 | 121 |
| 1122 | February 17 | 842 | 798 | 106 |
| 1122 | March 1 | 1127 | 880 | 128 |
| 1122 | February 12 | 825 | 824 | 100 |
| | **TOTAL** | **7490** | **6719** | 111 |

As shown above in Table 7, tomato plants exhibited an increase in total weight of fruit production of up to 42% when PRBT was applied compared to untreated plants. Plants flowered earlier increasing the first harvest day yield.

### Example 6 - Watermelon plants treated with PRBT have an increased fruit production and quality

PRBT applied exogenously increases plant production in watermelon. Watermelon plants var. Motril in 2014 and Boston in 2016 were sown a greenhouse on December 19, 2014 and 2016. A total of 1600 plants were used, 800 control plants and 800 PRBT treated plants distributed in random blocks. PRBT (with surfactant 2:1.5 v/w in 2014; and with surfactant 2:1 v/w, antifoaming agent and biocide in 2016) treated plants were sprayed four times once per month, from January to April with (0.054 g L-1) PRBT at 0.36 ml/plant/ month and with spinosad in all treatments as well as with mancozeb in the last treatment. The control plants received the spinosad and mancozeb treatments without the PRBT. The plants were harvested once in 2014 and twice in 2016 in April and production data were collected per harvest day according to the category, with category 1 (CAT1) being the best and category 2 (CAT2) being of less quality.

**Table 8 - Total weight watermelon production for PRBT spray treated watermelon plants per category and harvest date**

| | PRBT | | Control | |
|---|---|---|---|---|
| Date | Total Kg CAT1 production | Total Kg CAT2 production | Total Kg CAT1 production | Total Kg CAT1 production |
| 2014, April 8 | 16099 | 1533 | 10072 | 1402 |
| Total | 17632 | | 11474 | |
| 2016, April 8 | 9063 | 86 | 3946 | 157 |
| 2016, April 15 | 4102 | 0 | 4005 | 1486 |
| Total | 13251 | | 9594 | |

As shown above in **Table 8,** watermelon plants treated with PRBT increased total fruit production by 46.5% and increased fruit quality by 62% when compared to untreated plants, since only 5.2% of the PRBT treated plants where of Category 2, compared to the 14.4% of the untreated plants.

**Table 9 - Percentage of CAT1 production for PRBT spray treated and control watermelon plants per year**

| Year | % CAT1 production per PRBT plants | % CAT1 production per CONTROL plants |
|---|---|---|
| 2014 | 91.3 | 87.8 |
| 2016 | 99.4 | 82.9 |

As shown above in **Table 9,** watermelon plants treated with PRBT increased fruit quality when compared to untreated plants.

### Example 7 - Pepper plants treated with PRBT have an increased fruit production and quality

PRBT applied exogenously increases plant production in pepper. Pepper plants var. California or Guepard were sown in three independent greenhouses and fruits collected in 10 harvests from 64 plants per greenhouse randomly distributed. Plants were grown between January 20, 2014 and October 23, 2014. A total of 512 plants were harvested per greenhouse, 256 control plants and 256 PRBT (with surfactant 2:1.5 v/w) treated plants distributed in random blocks. PRBT treated plants were sprayed nine times, once per month from January to April with (0.054 g L-1) PRBT at 0.36 ml/plant/month.

**Table 10 - Total production per pepper plant in grams for PRBT spray or control treated plants * Indicates statistical significant differences between control and PRBT groups (α=0.05)**

| **Greenhouse/Variety** | **GROUP** | **PRODUCTION PER PLANT (grams)** | | |
|---|---|---|---|---|
| | | PROD. PER PLANT | **GAIN** | **%** |
| 1/California | CONTROL | 1001.51 ± 63.54 | + **337.23g*** | **+ 33.7%*** |
| | PRBT | 1338.74 ± 43.08 | | |
| 2/California | CONTROL | 516.29 ± 20.04 | + **126.24g*** | **+ 22.5%*** |
| | PRBT | 632.532 ± 19.92 | | |
| 3/Guepard | CONTROL | 915.56 ± 49.52 | + **228.81g*** | + **25%*** |
| | PRBT | 1138.38 ± 79.76 | | |

As shown above in **Table 10,** pepper plants treated with PRBT had an increase of fruit production between 22.5% and 33.7% compared to untreated plants.

**Table 11 - Number of fruits per pepper plant in grams for PRBT spray or control treated plants * Indicates statistical significant differences between control and PRBT groups (α=0.05)**

| **Greenhouse/Variety** | **GROUP** | **NUMBER OF FRUITS PER PLANT** | | |
|---|---|---|---|---|
| | | FRUITS PER PLANT | GAIN | % |
| 1/California | CONTROL | 5.34 ± 0.33 | + **0.97*** | **+18.1%*** |
| | PRBT | 6.31 ± 0.23 | | |
| 2/California | CONTROL | 3.87 ± 0.11 | + **0.5*** | **+ 12.9%*** |
| | PRBT | 4.37 ± 0.11 | | |
| 3/Guepard | CONTROL | 5.03 ± 0.29 | + **1.10*** | **+ 21.9%*** |
| | PRBT | 6.13 ± 0.29 | | |

As shown above in Table 11, pepper plants treated with PRBT had an increase in the total number of fruits per plant of between 12.9% and 21.9% compared to untreated plants.

**Table 12 - Weight per pepper fruit in grams for PRBT spray or control treated plants**

| **Greenhouse/Variety** | **GROUP** | **WEIGHT OF FRUITS (grams)** | | |
|---|---|---|---|---|
| | | AVERAGE WEIGHT | GAIN | % |
| 1/California | CONTROL | 185.97 ± 3.15 | + **26.10g*** | + **14.0%*** |
| | PRBT | 212.08 ± 2.49 | | |
| 2/California | CONTROL | 179.54 ± 163 | + **6.27g*** | **+ 3.48%*** |
| | PRBT | 185.81 ± 1.60 | | |

| | | | | |
|---|---|---|---|---|
| * Indicates statistical significant differences between control and PRBT groups (α=0.05). | | | | |

As shown above in **Table 12,** pepper plants treated with PRBT had an increase in the average weight of fruit between 3.48% and 14% compared to untreated plants.

### Example 8 - Soybean plants treated with PRBT have an increased tolerance against white mold (Sclerotinia sclerotiorum)

Soybean plants were sown on September 16, 2016 and distributed in random blocks of 9 plants with 4 replicates (36 plants per treatment) and grown in a standard greenhouse. PRBT (with surfactant 2:1 v/w, antifoam, and biocide) treated plants were sprayed once on October 25, with (0.054 g L⁻¹) PRBT at 6 ml/plant. Two days later plants were inoculated with 10 ml/plant of a foliar spray of blended *Sclerotinia sclerotiorum* strain CH109 mycelium that had been grown to optical density at 600 nm of 1.5 (in the C1 inoculated plants) or 2.0 in the C2 inoculated plants). Plants were kept at 100% relative humidity for the rest of the experiment. Plants were then evaluated 7, 13, and 18 days post inoculation (dpi) according to a disease severity index score where 0 corresponds to no symptoms and 5 corresponds to a dead plant. The area under the disease progress curve (AUDPC) was calculated as a quantitative summary of disease intensity over time.

**Table 13 - Disease index and AUDPC for control plants and plants inoculated with two concentrations of white mold and treated with PRBT**

| TREATMENT | 7dpi | 13 dpi | 18 dpi | AUDPC |
|---|---|---|---|---|
| CONTROL | 0.1 c | 0.3 d | 0.5 c | 3.42d |
| Inoculated C1 Untreated | 2.6 a | 2.6 ab | 2.8 ab | 29.33 ab |
| Inoculated C2 Untreated | 2.7 a | 2.8 a | 3.0 a | 31.03 a |
| Inoculated C1 PRBT | 1.8 b | 2.1 c | 2.5 b | 23.54 c |
| Inoculated C2 PRBT | 1.9 b | 2.2 bc | 2.6 b | 24.42 c |

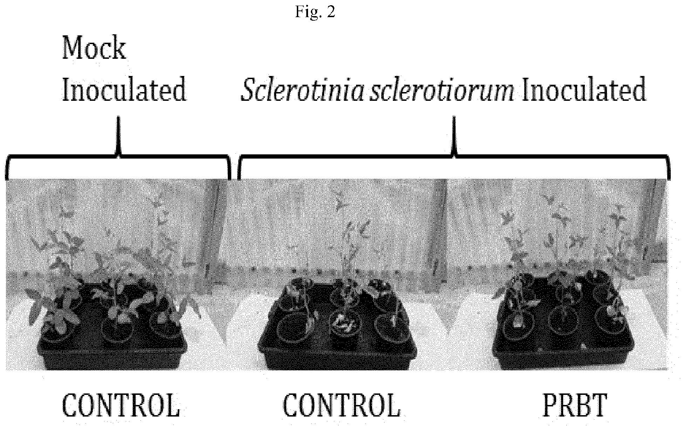

As shown above in **Table 13** the disease index in PRBT treated soybean plants was significantly lower than in untreated soybean plants that had been inoculated with two different *Sclerotinia sclerotiorum* mycelium doses. No statistically significant differences among treatments with the same letter within the same column (LSD method, p= 0.05). For example, at 13 dpi, the inoculated C2 untreated is significantly different from the inoculated C1 PRBT treated (2.8 a versus 2.1 c), however, there is no significant difference between inoculated C1 and C2 PRBT plants (2.1 c versus 2.2 bc). Figure 2 shows photographs of soybean plants inoculated with *Sclerotinia sclerotiorum,* (C1) and treated with PRBT (right panel) compared to *Sclerotinia sclerotiorum* (C1) inoculated control plants (middle panel) and mock inoculated control treated plants (left panel).

### Example 9 - Tomato plants treated with PRBT have an increased tolerance against white mold (Sclerotinia sclerotiorum) and increased fruit production under infection conditions

Tomato plants var. Royesta were sown on January 28, 2014 and distributed in random blocks of 5 plants with 6 replicates (30 plants per treatment) and grown in a standard production greenhouse. PRBT treated plants were sprayed twice on February 18, and on March 11^{th} with (0.054 g L⁻¹) PRBT (without surfactant) at 6 ml/plant or with stemicol (4.5g L⁻¹) at 6 ml/plant. Two days later plants were inoculated with 20 ml/plant of a foliar spray of blended *Sclerotinia sclerotiorum* strain CH109 mycelium that had been grown to optical density at 600 nm of 1.7. Plants were kept at 100% relative humidity for ten days and then humidity was lowered to 80% for the rest of the experiment. Plants were then evaluated 11, 18, 41 and 55 days post inoculation (dpi) according to a disease severity index score where 0 corresponds to no symptoms and 5 corresponds to a dead plant. The tomatoes were harvested at 90 dpi and total weight per plant recorded.

**Table 14 - Disease index and total production per plant in kilograms for PRBT spray, stemicol or control treated plants inoculated with of white mold and treated with PRBT**

| TREATMENT | 11dpi | 18dpi | 41 dpi | 55 dpi | Total fruit Kg per plant | % Control |
|---|---|---|---|---|---|---|
| CONTROL | 0.0 c | 0.0 d | 0.8 c | 1.0 c | 6.5d | 100 |
| Inoculated Untreated | 2.0 a | 2.4 a | 3.1 a | 3.7 a | 1.1a | 16.9 |
| Inoculated stemicol | 1.6 b | 1.9 b | 2.3 b | 2.7 b | 2.4b | 36.9 |
| Inoculated PRBT | 1.6 b | 1.6 c | 2.3 b | 2.5 b | 3.5c | 53.8 |

As shown above in **Table 14** above the disease index in PRBT treated tomato plants was significantly lower than in untreated tomato plants and equivalent to stemicol treated plants that had been inoculated *Sclerotinia sclerotiorum.* Furthermore, the lower disease index correlated with an increase of total fruit production per plant 36.9% compared to untreated inoculated plants and 16.9% compared to stemicol treated inoculated plants. No statistically significant differences among treatments with the same letter (LSD method, p= 0.05). For example, at 18 dpi the plants inoculated and treated with PRBT had a significantly lower disease index (1.6 c) compared to plants treated with stemicol (1.9 b) and untreated (2.4 a)

### Example 10 - Pepper plants treated with PRBT have an increased tolerance against Botrytis cinerea and increased fruit production under infection conditions

Pepper plants var. Ferrari were sown on November 26, 2014 and distributed in random blocks of 5 plants with 6 replicates (30 plants per treatment) and grown in a standard production greenhouse. PRBT (with surfactant 2:1 v/w) treated plants were sprayed on January 13, 2015 with (0.054 g L⁻¹) PRBT at 20 ml/plant or with stemicol (4.5g L⁻¹) at 20 ml/plant. One day later plants were inoculated with 20 ml/plant of a foliar spray of 10⁶ conidia/ml of *Botrytis cinerea* strain CH98. Plants were kept at 100% relative humidity for ten days and then humidity was lowered to 80% for the rest of the experiment. Plants were then evaluated 22, 49, and 58 days post inoculation (dpi) according to according to a disease severity index score where 0 corresponds to no symptoms and 5 corresponds to a dead plant. The peppers were harvested at 122, 135, 150, 170 and 200 dpi and total weight per plant recorded.

**Table 15 - Disease index and total production per plant in kilograms for PRBT spray, stemicol or control treated plants inoculated with of white mold and treated with PRBT**

| TREATMENT | 22dpi | 49dpi | 58 dpi | Total fruit Kg per plant | % Control |
|---|---|---|---|---|---|
| CONTROL | 0.45 b | 1.0 c | 1.45 b | 3.1b | 100 |
| Inoculated Untreated | 1.95 a | 2.0 a | 2.05 a | 1.6a | 51.6 |
| Inoculated stemicol | 1.65 a | 1.6 bc | 1.7 ab | 1.6a | 51.6 |
| Inoculated PRBT | 1.35 a | 1.35 bc | 1.45 b | 2.7b | 87.1 |

As shown above in **Table 15** above the disease index in PRBT treated pepper plants was significantly lower than in untreated pepper plants and equivalent to stemicol treated plants that had been inoculated *Botrytis cinerea.* Furthermore, the lower disease index correlated with an increase of total fruit production per plant 35.5% compared to untreated inoculated plants and to stemicol treated inoculated plants. No statistically significant differences among treatments with the same letter (LSD method, p= 0.05). For example, at 49 dpi, plants inoculated and treated with PRBT had a significantly lower disease index (1.35 bc) compared to inoculated, untreated plants (2.0 a).

### Example 11 - Tomato plants treated with PRBT and Bacillus subtilis have increased tolerance against white mold (Sclerotinia sclerotiorum)

Tomato plants var. Ventero were sown on April 5, 2016 and distributed in random blocks of 9 plants with 7 replicates (63 plants per treatment) and grown in a standard greenhouse. PRBT (with surfactant 2:1.5 v/v and antifoam), *Bacillus subtilis* or a combination of both was sprayed three times before inoculation on June 20, July 7, and July 20, with (0.038 g L⁻¹) PRBT (C1), or (0.054 g L⁻¹) PRBT (C2) and/or *Bacillus subtilis* at 4 l/ha (C3), or, *Bacillus subtilis* at 6 l/ha (C4). On July 22^{nd} plants were inoculated with 10 ml/plant of a foliar spray of blended *Sclerotinia sclerotiorum* strain CH109 mycelium that had been grown to optical density at 600 nm of 1.3. Plants were kept at 100% relative humidity for ten days and then humidity was lowered to 80% for the rest of the experiment. Plants were then evaluated 7, 14, 21 and 35 days post inoculation (dpi) according to a disease severity index score were 0 corresponds to no symptoms and 10 to a dead plant. The area under the disease progress curve (AUDPC) was calculated as a quantitative summary of disease intensity over time.

**Table 16 - Disease index and AUDPC for control plants and plants inoculated with two concentrations of white mold and treated with PRBT**

| TREATMENT | 7dpi | 14 dpi | 21 dpi | 35 dpi | AUDPC |
|---|---|---|---|---|---|
| CONTROL | 0.0 c | 0.0 d | 0.0 c | 0.0 c | 0.0 d |
| Inoculated Untreated | 2.9 a | 3.8 a | 5.7 ab | 5.9 ab | 126.3 a |
| Inoculated C1 PRBT | 1.7 b | 2.3 bc | 3.7 bc | 4.0 bc | 81.3 bc |
| Inoculated C2 PRBT | 1.7 b | 2.2 bc | 4.0 bc | 4.1 bc | 84.2 bc |
| Inoculated C1 PRBT+ C3 *Bacillus subtilis* | 1.6 b | 2.3 bc | 3.8 bc | 3.9 bc | 80.9 bc |
| Inoculated C2 PRBT+ C4 *Bacillus subtilis* | 1.6 b | 2.1 c | 3.1 c | 3.3 c | 70.2 c |
| C3 *Bacillus subtilis* | 2.0 b | 2.9 b | 4.3 b | 4.6 b | 95.9 b |
| C4 *Bacillus subtilis* | 2.1 b | 2.8 bc | 4.3 b | 4.6 b | 95.9 b |

| | | | | | |
|---|---|---|---|---|---|
| "C1 PRBT" = 0.038 g L⁻¹ of the PRBT with surfactant and antifoam "C2 PRBT" = 0.054 g L⁻¹ of the PRBT with surfactant and antifoam "C3 PRBT" = *Bacillus subtilis* at 4 l/ha "C4 PRBT" = *Bacillus subtilis* at 61/ha | | | | | |

As shown above in **Table 16** the disease index and AUDPC in tomato plants treated with two concentrations of PRBT or with two concentrations of *Bacillus subtilis* were significantly lower than in untreated tomato plants inoculated with *Sclerotinia sclerotiorum.* Furthermore, the disease index and AUDPC in tomato plants, treated with a combination of two concentrations of PRBT and *Bacillus subtilis*, were significantly lower than in untreated tomato plants that had been inoculated with *Sclerotinia sclerotiorum.* At the highest concentration combination of PRBT (C2) and *Bacillus subtilis* (C4) the disease index and AUDPC in *Sclerotinia sclerotiorum* inoculated tomato plants were significantly lower than in tomato plants treated only with a low (C3) or high (C4) concentration of *Bacillus subtilis.* No statistically significant differences among treatments with the same letter (LSD method, p= 0.05). For example, at 21 dpi, plants inoculated and treated with C2 PRBT + C4 *Bacillus subtilis* had a significantly lower disease index (3.1 c) compared to plants inoculated and treated with C3 *Bacillus subtilis* (4.3). There was no difference between C3 and C4 *Bacillus subtilis* (4.3 b and 4.3 b, respectively).

### Example 12 - Soybean plants treated with PRBT and Bacillus subtilis have increased tolerance against white mold (Sclerotinia sclerotiorum)

Soybean plants were sown on and distributed in random blocks of 9 plants with 7 replicates (63 plants per treatment) and grown in a standard greenhouse. PRBT (with surfactant 2:1.5 v/v and antifoam), *Bacillus subtilis* or a combination of both was sprayed once time before inoculation on with (0.038 g L⁻¹) PRBT (C1), or (0.054 g L⁻¹) PRBT (C2) and/or *Bacillus subtilis* at 4 l/ha (C3), or, *Bacillus subtilis* at 6 l/ha (C4). Plants were inoculated with 10 ml/plant of a foliar spray of blended *Sclerotinia sclerotiorum* strain CH109 mycelium that had been grown to optical density at 600 nm of 1.3. Plants were kept at 100% relative humidity for ten days and then humidity was lowered to 80% for the rest of the experiment. Plants were then evaluated for 6, 9, and 14 days post inoculation (dpi) according to a disease severity index score where 0 corresponds to no symptoms and 5 corresponds to a dead plant. The area under the disease progress curve (AUDPC) was calculated as a quantitative summary of disease intensity over time as well as the efficacy of protection.

**Table 17 - Disease index AUDPC for control plants and plants inoculated with two concentrations of white mold and treated with PRBT**

| TREATMENT | 6dpi | 9 dpi | 14 dpi | AUDPC | Efficacy |
|---|---|---|---|---|---|
| CONTROL | 0.0 e | 0.0 e | 0.0 d | 0.0 d | 100 a |
| Inoculated Untreated | 3.3 a | 3.4 a | 3.6 a | 27.6 a | 0.0 d |
| Inoculated C1 PRBT | 2.5 bc | 2.9 b | 2.9 b | 22.7 b | 17.7 c |
| Inoculated C2 PRBT | 2.3 cd | 2.6 cd | 2.6 c | 20.0 c | 27.6 b |
| Inoculated C1 PRBT + C3 *Bacillus subtilis* | 2.0 d | 2.4 d | 2.5 c | 19.0 c | 31.0 b |
| Inoculated C2 PRBT + C4 *Bacillus subtilis* | 2.0 d | 2.5 d | 2.5 c | 19.1 c | 30.9 b |
| C3 *Bacillus subtilis* | 2.6 bc | 2.8 bc | 2.8 b | 22.2 b | 19.4 c |
| C4 *Bacillus subtilis* | 2.7 b | 3.0 b | 3.0 b | 23.6 b | 14.1 c |

As shown above in **Table 17** the disease index, AUDPC and efficacy in soybean plants treated with two concentrations of PRBT or with two concentrations of *Bacillus subtilis* were significantly lower than in untreated soybean plants inoculated with *Sclerotinia sclerotiorum*. Furthermore, the disease index, AUDPC and efficacy in soybean plants, treated with a combination of two concentrations of PRBT and *Bacillus subtilis*, were significantly lower than in untreated soybean plants inoculated with *Sclerotinia sclerotiorum.* The disease index, AUDPC and efficacy in soybean plants, treated with a combination of two concentrations of PRBT and *Bacillus subtilis*, were also significantly lower than in soybean plants, treated only with a low (C3) or high (C4) concentration of *Bacillus subtilis*, or with a low (C1) concentration of PRBT alone. No statistically significant differences among treatments with the same letter (LSD method, p= 0.05). For example, at 9 dpi, plants inoculated and treated with C1 PRBT had a significantly lower disease index (2.9 b) compared to plants inoculated and untreated (3.4 a).

### Example 13 - Zucchini plants treated with PRBT have an increased tolerance against tomato leaf curl New Dehli virus (ToLCNDV).

Natural ToLCNDV infection occurred in zucchini plants var. Victoria and Cronos grown under standard greenhouse conditions by a experimented farmer in Almería, Spain. In total, 2200 (var. victoria) and 4300 (var. cronos) plants were used as untreated controls whereas 4400 (var. Victoria) and 2190 (var. Cronos) plants were sprayed every two weeks with PRBT (0.038g L⁻¹) in combination with abamectine twice and with either imidacloprod or spinosad alternating every other week. Furthermore, mancozeb was also added as a combination twice in 2016. The 2016 PRBT contained antifoam and a biocide as well as the surfactant. In 2014 and 2015 plants were sprayed once a month with PRBT (0.054 g L⁻¹) in combination with spinosad. The 2014 PRBT contained the surfactant in a 2:1.5 v/v proportion, while the 2015 PRBT contained surfactant in a 2:1 v/w proportion. Control plants were treated with abamectine or imidacloprod or spinosad or mancoceb alone. To detect viral load, completely randomized blocks of 10 plants with 18 replicates (180 plants for each control and PRBT-treated) were designed and 2 young leaves/plant were harvested and used for tissue print hybridization with a virus specific Digoxigenin-labeled probe on positively charged nylon membranes. The Digoxigenin-labeled probe was obtained by PCR amplification from the partial AV1 gene from DNA-A of ToLCNDV using the primer pairs ToNDA-580F:5'-TCACACATCGCGTAGGCAAG-3' (SEQ ID NO:1) and ToNDA-935R: 5'-TGCCGGCCTCTTGTTGATTG-3' (SEQ ID NO:2) with the PCR DIG Labeling Mix (Roche Diagnostics, Switzerland) and following the manufacturer's instructions. Immunodetection was performed with anti-digoxigenin antibody conjugated with alkaline phosphatase (Roche Diagnostics, Switzerland) and chemiluminescence with CSPD (Roche Diagnostics, Switzerland) as substrate, following the manufacturer's instructions and different times of exposure (15 min -overnight) to a Lumi-film (Amersham Bioscience, UK). Virus disease index was calculated number of TolCNDV symptomatic plants/total number of plants for 2014 and 2015 or number of TolCNDV positive plants/180 total number of sampled plants for 2016.

**Table 18 - ToLCNDV infection index in PRBT or untreated zucchini plants**

| Treatments | Viral infection index 2014 | Viral infection index 2015 | Viral infection index 2016 | T2 P-value |
|---|---|---|---|---|
| Untreated | 17.7% | 6.65% | 16.21% | 0.00124139 |
| PRBT | 6.7% | 1.89% | 5.82% | |

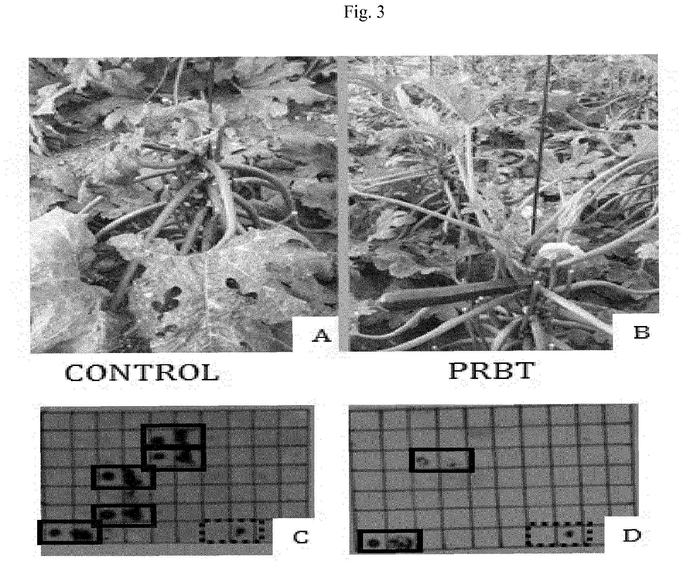

As shown above in **Table 18,** plants treated with PRBT had a lower infection index by tomato leaf curl New Dehli virus (ToLCNDV). Figure 3, shows photographs of zucchini plants infected with ToLCNDV treated with PRBT (panel B) compared to control (untreated) plants (panel A). Inmunodetection membranes of 34 zucchini plants treated with PRBT (panel D) compared to control (untreated) plants (panel C) are shown where infected plants with tomato leaf curl New Dehli virus are highlighted with a solid line rectangle with tissue prints of the stem (left) and leaf (right) while negative (left) and positive controls (right) are indicated with a discontinuous rectangle. The ToLCNDV infected control (untreated) plants (panel C) show a higher virus load in both stem and leaves than the PRBT treated plants (panel D).

**Table 19** below shows the total zucchini fruit production per plant in treated and untreated plants. Treated plants were sprayed every four weeks with 0.054 grams per liter PRBT or every two weeks with 0.038 grams per liter PRBT. Column 1 shows the season, column 2 shows the group, column 3 shows the average harvested production for each plant in kilograms, column 4 shows the number of plants for each group, column 5 shows the percentage of gain with respect to the control values, and column 6 shows the P-value corresponding to statistical T2 analysis for daily production per plant data and treatment. P-values lower than 0.05 indicate significant differences between control and treated groups (α=0.05). The plants were grown in a conventional greenhouse by an experimented farmer during the last 3 seasons in Almeria, Spain. Percentage respect to the control values is shown for each year harvest data.

**Table 19 - Total zucchini fruit production per plant (Kg.) sprayed every four weeks with PRBT (0.054g per liter) once per month or every two weeks with PRBT (0.038g per liter) twice per month and non-treated (control) plants**

| SEASON | TREATMENT | PRODUCTION PER PLANT (Kg.) | *N* | % GAIN | T² P-VALUE |
|---|---|---|---|---|---|
| WINTER 2014 | CONTROL | 7.10 | 4300 | 100 | 0.000235332 |
| | PRBT | 9.82 | 2190 | 138.3 | |
| SUMMER 2015 | CONTROL | 3.03 | 2200 | 100 | |
| | PRBT | 3.53 | 4400 | 116.4 | |
| WINTER 2016 | CONTROL | 6.89 | 2300 | 100 | |
| | PRBT | 7.23 | 4600 | 104.9 | |

As shown above in **Table 19,** plants treated with PRBT showed between 4.9% and 38.3% increased fruit production compared to untreated plants.

### Example 14 - PRBT contains 6-oxo-piperidine-2-carboxylic acid and not 6-hydroxypiperidine-2-carboxylic acid.

The PRBT 6-hydroxypiridine-2-carboxylic acid and 6-oxo-piperidine-2-carboxylic acid content was determined by quantitative MRM (LC-QQQ-MS). A total of 75 µL of PRBT triplicate samples were dissolved in 100 mL of water, filtered, and analyzed by LC-MS with an Injection volume of 20 µL at a flow of 0.4 mL/min and running time of 22 min (excluding 11 min wash between samples). The MRM Transitions were: 6-hydroxypiridine-2-carboxilic acid, Quantifier (m/z): 138.10 > 93.95 CE: +13, Qualifier (m/z): 138.10 > 40.10 CE: +36, 6-oxo-piperidinil acid, Quantifier (m/z): 144.1 > 97.95 CE: -15, Qualifier (m/z): 144.1 > 55.05 CE: -26. The Analytical Column was a ZORBAX RX-SIL 5u 110 A 150 x 2.1mm.

**Table 20. 6-oxopiperidine-2-carboxylic acid and 6-hydroxypiperidine-2-carboxylic acid content in three samples of PRBT**

| Sample | Concentration 6-hydroxypiperidine-2-carboxylic acid (mg/L) | Concentration 6-oxopiperidine-2-carboxylic acid (mg/L) |
|---|---|---|
| PRBT 1 | ND | 2107.3 |
| PRBT 2 | ND | 2289.4 |
| PRBT 3 | ND | 1669.4 |

As shown above in **Table 20**, PRBT does not contain 6-hydroxypiridin-2-carboxilic acid, while it contains an average of 2022 ± 15.8 mg/L of 6-oxopiperidine-2-carboxylic acid.

While a number of exemplary aspects and embodiments have been discussed above, those of skill in the art will recognize certain modifications, permutations, additions and sub-combinations thereof. It is therefore intended that the following appended claims and claims hereafter introduced are interpreted to include all such modifications, permutations, additions, and sub-combinations as are within their true spirit and scope.

### Example 15 - Tomato plants treated with 6-oxopiperidine-2-carboxylic acid or 6-hydroxypiperidine-2-carboxylic acid have an increased tolerance against white mold (Sclerotinia sclerotiorum)

Tomato plants var. Money Maker were sown on December 13, 2016, distributed in random blocks of 9 plants with 4 replicates (36 plants per treatment) and grown in a standard greenhouse. Treated plants were sprayed on March 6, 2017 with 25 ml /plant at 3 ml/l (0.054 g l⁻¹) of PRBT(with surfactant 2:1, v/w, antifoam and biocide) or with 25 ml (0.1g l⁻¹) of 6-hydroxypiperidine-2-carboxylic acid (6-HP-2CA), or with 25 ml (0.1g l-1) of 6-oxopiperidine-2-carboxylic acid (6-OXO-2CA).
Two days later plants were inoculated with 25 ml/plant of a foliar spray of blended *Sclerotinia sclerotiorum* strain CH109 mycelium that had been grown to optical density at 600 nm of 0.8. Plants were kept at 100% relative humidity for seven days and then humidity was lowered to 80% for the rest of the experiment. Plants were then evaluated at 10 and 14 days post inoculation (dpi) according to a disease severity index score were 0 corresponds to no symptoms and 5 to a dead plant. No statistically significant differences among treatments with the same letter (LSD method, p= 0.05)

**Table 21 - Disease index for PRBT spray, 6-hydroxypiperidine-2-carboxylic acid, 6-oxopiperidine-2-carboxylic acid treated plants and untreated plants inoculated with white mold.**

| TREATMENT | Disease index (10 dpi) | Disease index (15 dpi) | Disease Control (%) |
|---|---|---|---|
| CONTROL | 0.0 a | 0.0 a | 100 a |
| Inoculated Untreated | 2.6 a | 3.1 a | 0 d |
| Inoculated PRBT | 1.4 c | 1.4 c | 35 b |
| Inoculated 6-HP-2CA 0.1 g/l | 2.2 b | 2.2 b | 11 c |
| Inoculated 6-OXO-2CA 0.1 g/l | 2.3 b | 2.3 b | 10 c |

As shown above in Table 21 the disease index in tomato plants treated with PRBT, or with 6-HP-2CA (0.1 g/l) or with 6-OXO-2CA was significantly lower than in untreated tomato plants that had been inoculated with *Sclerotinia sclerotiorum.* No statistically significant differences among treatments with the same letter (LSD method, p= 0.05)

### Example 15 - Pepper plants treated with 6-hydroxypiperidine-2-carboxylic acid have an increased tolerance against white mold (Sclerotinia sclerotiorum)

Pepper plants var. Murano were sown on October 20, 2016 and distributed in random blocks of 3 plants with 4 replicates (12 plants per treatment) and grown in a standard greenhouse. PRBT treated plants were sprayed on November 23, 2016 with (0.038 g L⁻¹) PRBT at 10 ml/plant or with (0.01g L⁻¹) 6-hydroxypiperidine-2-carboxylic acid (6-HP-2CA) or with (0.1g L⁻¹) 6-HP-2CA or with (0.01g L⁻¹) 6-HP-2CA formulated with an adjuvant or with (0.1g L⁻¹) 6-HP-2CA (0.01g L⁻¹) formulated with an adjuvant at 10 ml/plant. Two days later plants were inoculated with 10 ml/plant of a foliar spray of blended *Sclerotinia sclerotiorum* strain CH109 mycelium that had been grown to optical density at 600 nm of 1.7. Plants were kept at 100% relative humidity for four days and then humidity was lowered to 80% for the rest of the experiment. Plants were then evaluated 4, and 6 days post inoculation (dpi) according to a disease severity index score were 0 corresponds to no symptoms and 4 to a dead plant. No statistically significant differences among treatments with the same letter (LSD method, p= 0.05)

**Table 22 - Disease index for PRBT spray, 6-hydroxypiperidine-2-carboxylic acid (6-HP-2CA) or control treated plants inoculated with white mold**

| TREATMENT | 4 dpi | 6 dpi |
|---|---|---|
| CONTROL | 0.0 a | 0.0 a |
| Inoculated Untreated | 1.1 b | 2.5 b |
| Inoculated PRBT | 0.2 a | 0.6 a |
| Inoculated 6-HP-2CA 0.01 g/L | 0.9 b | 2.3 b |
| Inoculated 6-HP-2CA 0.1 g/L | 0.2 a | 0.5 a |
| Inoculated 6-HP-2CA 0.01 g/L Formulated | 0.0 a | 0.8 a |
| Inoculated 6-HP-2CA 0.1 g/L Formulated | 0.0 a | 0.1 a |

As shown above in **Table 22** the disease index in pepper plants treated with PRBT, or with a high (0.1 g/L), or a low (0.01 g/L) concentration of formulated 6-HP-2CA, or with a high (0.1 g/L) concentration of 6-HP-2CA was significantly lower than in untreated pepper plants that had been inoculated with *Sclerotinia sclerotiorum.* No statistically significant differences among treatments with the same letter (LSD method, p= 0.05). For example, at 6 dpi, plants inoculated and treated with PRBT had a significantly lower disease index (0.6 a) compared to inoculated and untreated plants (2.5 b).

The foregoing discussion of the disclosure has been presented for purposes of illustration and description. The foregoing is not intended to limit the disclosure to the form or forms disclosed herein. In the foregoing Detailed Description for example, various features of the disclosure are grouped together in one or more embodiments for the purpose of streamlining the disclosure. This method of disclosure is not to be interpreted as reflecting an intention that the claimed disclosure requires more features than are expressly recited in each claim. Rather, as the following claims reflect, inventive aspects lie in less than all features of a single foregoing disclosed embodiment. Thus, the following claims are hereby incorporated into this Detailed Description, with each claim standing on its own as a separate preferred embodiment of the disclosure.

## Claims

1. A composition for increasing a growth characteristic of a plant or part thereof, increasing nutrient use efficiency of a plant or part thereof, and/or increasing abiotic and/or biotic stress tolerance of a plant or part thereof stress comprising an effective amount of a fungal mycelia extract comprising piperidine and/or an analogue thereof, and/or a salt thereof, or any combination thereof.

2. The composition of claim 1, wherein the piperidine analogue and/or salt is 6-oxopiperidine-2-carboxylic acid and/or 6-oxopiperidine-2-carboxylate.

3. The composition of claim 1 or claim 2, wherein the fungal mycelial extract further comprises a peptide, a protein, a sugar, a carbohydrate, or any combination thereof.

4. The composition of any one of claims 1 to 3, wherein the composition further comprises a surfactant, a humectant, an adjuvant, an antioxidant, a preservative, a plant macronutrient, a plant micronutrient, a plant growth regulator, a pesticide, a fungicide, an antiviral, an anti-bacterial, a herbicide, or any combination thereof.

5. The composition of any one of claims 1 to 4, wherein the composition further comprises a beneficial microbe, optionally wherein the beneficial microbe is *Bacillus subtilis*, *Pseudomonas* spp, *Azotobacter* spp, *Azospirillum* spp, *Rhizobium* spp, *Azorhizobium* spp, *Chaetomium* spp, *Streptomyces* spp. *Trichoderma* spp., and/or mycorrhizal fungi.

6. The composition of any one of claims 1 to 5, wherein the composition is in the form of an aqueous solution, a non-aqueous solution, a suspension, a gel, a foam, a paste, a powder, a dust, a solid, and/or an emulsion.

7. The composition of any one of claims 1 to 6, wherein the piperidine and/or analogue thereof, or salt thereof is in the composition in an amount from about 0.1 grams per liter to about 50 g per liter of the composition.

8. A method for increasing a growth characteristic of a plant or part thereof, the method comprising
applying a composition comprising an effective amount of a fungal mycelia extract to a plant or plant part thereof, wherein the extract comprises piperidine and/or an analogue thereof, and/or a salt thereof, or any combination thereof, thereby increasing the growth characteristic of the plant or part thereof as compared to a control plant or part thereof.

9. A method for increasing nutrient use efficiency of a plant or part thereof, the method comprising
applying a composition comprising an effective amount of a fungal mycelia extract to a plant or plant part thereof, wherein the extract comprises piperidine and/or an analogue thereof, and/or a salt thereof, or any combination thereof, thereby increasing nutrient use efficiency of the plant or part thereof as compared to a control plant or part thereof.

10. The composition of claim 8 or claim 9, wherein the piperidine analogue and/or salt is 6-oxopiperidine-2-carboxylic acid and/or 6-oxopiperidine-2-carboxylate.

11. The method of any one of claims 8 to 10, wherein applying comprises contacting the plant with the composition at least two times.

12. The method of any one of claims 8 to 11, wherein the fungal mycelial extract further comprises a peptide, protein, a sugar, a carbohydrate, or any combination thereof.

13. The method of any one of claims 8 to 12, wherein the composition further comprises a surfactant, a humectant, an adjuvant, an antioxidant, a preservativer, a plant macronutrient, a plant micronutrient, a plant growth regulator, a pesticide, a fungicide, an antiviral, an anti-bacterial, a herbicide, or any combination thereof.

14. The method of any one of claims 8 to 13, wherein the effective amount of the extract in the composition is in a range from about 0.005 grams per liter to about 150 g per liter of the composition, optionally about 0.05 grams per liter to about 100 grams per liter, or about 1 gram per liter to about 50 grams per liter of the composition.

15. The method of any one of claims 8 or 10 to 14, wherein the increased growth characteristic is increased fruit production, increased inflorescence production, increased fruit quality, and/or an increased biomass as compared to a control plant or plant part thereof.

16. The method of any one of claims 8 to 15, wherein said plant is a monocotyledonous or dicotyledonous plant.

17. The method of any one of claims 8 to 16, wherein said plant part is a seed.

18. A method for increasing disease tolerance of a plant or part thereof, the method comprising
applying a composition comprising an effective amount of a fungal mycelia extract to a plant or plant part thereof, wherein the extract comprises piperidine and/or an analogue thereof, and/or a salt thereof, or any combination thereof; thereby increasing the disease tolerance of the plant or part thereof as compared to a control plant or part thereof.

19. A method for increasing abiotic stress tolerance of a plant or part thereof, the method comprising
applying a composition comprising an effective amount of a fungal mycelia extract to a plant or plant part thereof, wherein the extract comprises piperidine and/or an analogue thereof, and/or a salt thereof, or any combination thereof, thereby increasing the abiotic stress tolerance of the plant or part thereof as compared to a control plant or part thereof.

20. The composition of claim 18 or claim 19, wherein the piperidine analogue and/or salt is 6-oxopiperidine-2-carboxylic acid and/or 6-oxopiperidine-2-carboxylate.

21. The method of any one of claims 18 to 20, wherein applying comprises contacting the plant with the composition at least two times.

22. The method of any one of claims 18 to 21, wherein the fungal mycelial extract further comprises a peptide, a sugar, or any combination thereof.

23. The method of any one of claims 18 to 22, wherein the composition further comprises a surfactant, a humectant, an adjuvant, an antioxidant, a stabilizer, a plant macronutrient, a plant micronutrient, a plant growth regulator, a pesticide, a fungicide, an antiviral, an anti-bacterial, a herbicide, or any combination thereof.

24. The method of any one of claims 18 to 23, wherein said composition further comprises a beneficial microbe, optionally wherein the beneficial microbe is *Bacillus subtilis*, *Pseudomonas* spp, *Azotobacter* spp, *Azospirillum* spp, *Rhizobium* spp, *Azorhizobium* spp, *Chaetomium* spp, *Streptomyces* spp. *Trichoderma* spp., and/or mycorrhizal fungi.

25. The method of any one of claims 18 to 24, wherein the effective amount of extract in the composition is in a range from about 0.005 grams per liter to about 150 g per liter of the composition, optionally about 0.05 grams per liter to about 100 grams per liter, or about 1 gram per liter to about 50 grams per liter of the composition.

26. The method of any one of claims 18 or 20 to 25, wherein the disease to which the plant or part thereof has increased tolerance is a fungal disease.

27. The method of any one of claims 18 or 20 to 25, wherein the disease to which the plant or part thereof has increased tolerance is a viral disease.

28. The method of any one of claims 18 or 20 to 25, wherein the disease to which the plant or part thereof has increased tolerance is a bacterial disease.

29. The method of any one of claims 19 to 25, wherein the abiotic stress is stress due to salinity, drought, flooding, freezing, cold temperature, and/or high temperature.
